# FASCICULE DE BREVET EUROPEEN

(11) **EP 1 778 713 B1**
(45) Date de publication et mention de la délivrance du brevet: **31.12.2008**
(21) Numéro de dépôt: 05792074.6
(22) Date de dépôt: 21.07.2005
(51) Int. Cl.: C07H 21/00, C07H 1/08, C12Q 1/68, C07D 495/08

(54) **PROCEDE DE MARQUAGE ET DE PURIFICATION D'ACIDES NUCLEIQUES D'INTERET PRESENTS DANS UN ECHANTILLON BIOLOGIQUE A TRAITER DANS UN UNIQUE RECIPIENT REACTIONNEL**
VERFAHREN ZUR KENNZEICHNUNG UND REINIGUNG VON INTERESSIERENDEN NUKLEINSÄUREN IN EINER BIOLOGISCHEN PROBE ZUR BEHANDLUNG IN EINEM EINZELREAKTIONSGEFÄSS
METHOD FOR LABELLING AND PURIFYING NUCLEIC ACIDS OF INTEREST PRESENT IN A BIOLOGICAL SAMPLE TO BE TREATED IN A SINGLE REACTION VESSEL

(30) Priorité: 23.07.2004 FR 0451632
(43) Date de publication de la demande: 02.05.2007
(73) Titulaire: BIOMERIEUX, 69280 Marcy-L'Etoile (FR)
(72) Inventeur: LAAYOUN, Ali, F-38690 (FR); MENOU, Lionel, F-69230 SAINT GENIS LAVAL (FR); GINOT, Frédéric, F-38120 SAINT-EGREVE (FR)
(86) Numéro de dépôt international: PCT/FR2005/050601
(87) Numéro de publication internationale: WO 2006/018572

(56) Documents cités:
- WO-A-02/090584
- US-A- 4 775 745
- US-B1- 6 617 138
- LAAYOUN A ET AL: "Aryldiazomethanes for Universal Labeling of Nucleic Acids and Analysis on DNA Chips" BIOCONJUGATE CHEMISTRY, AMERICAN CHEMICAL SOCIETY, WASHINGTON, US, vol. 14, 2003, pages 1298-1306, XP002308387 ISSN: 1043-1802

## Description

La présente invention concerne un procédé de marquage d'acides nucléiques en présence d'au moins un support solide.

L'état de la technique montre que de nombreuses méthodes existent pour marquer des nucléotides, des oligonucléotides ou des acides nucléiques.

Une première méthode consiste à fixer le marqueur sur la base, que celle-ci soit naturelle ou modifiée. Une deuxième méthode propose de fixer le marqueur sur le sucre, là encore qu'il soit naturel ou modifié. Une troisième méthode a pour objet la fixation du marqueur sur le phosphate.

Le marquage sur la base a été notamment utilisé dans l'approche de marquage des acides nucléiques par incorporation de nucléotides directement marqués.

Le marquage sur le sucre est souvent utilisé dans le cas des sondes nucléiques préparées par synthèse chimique.

Le marquage sur le phosphate a été aussi utilisé pour introduire des bras fonctionnalisés et des marqueurs lors de la synthèse chimique des oligonucléotides.

En fait l'homme du métier, qui doit effectuer un marquage d'un nucléotide, ou d'un analogue de nucléotide ou d'un acide nucléique, est enclin à effectuer cette fixation sur la base ou sur le sucre qui lui offrent plus de commodité et d'alternatives. C'est d'ailleurs ce qui ressort de l'étude de nombreux documents, tels que EP-A-0.329.198, EP-A-0.302.175, EP-A-0.097.373, EP-A-0.063.879, US-A-5,449,767, US-A-5,328,824, WO-A-93/16O94, DE-A-3.910.151, EP-A-0.567.841 pour la base ou EP-A-0.286.898 pour le sucre.

La fixation du marqueur sur le phosphate est une technique plus complexe que la technique consistant à fonctionnaliser la base ou le sucre et a été bien moins utilisée notamment à cause de la faible réactivité du phosphate (voir par exemple Jencks W.P. et al J. Amer. Chem Soc., 82, 1778-1785, 1960). De même dans la revue de O'Donnel et Mc Laughlin (« Reporter groups for the analysis of nucleic acid structure », p 216-243, dans « Bioorganic Chemistry : Nucleic Acids », Ed Hecht S.M., Oxford University Press, 1996) portant sur les méthodes d'introduction de sondes dans les fragments d'oligonucléotides, l'alkylation efficace du phosphodiester intemucléotidique est considérée comme étant impossible.

La demande de brevet WO-A-99/65926 décrit un procédé de marquage d'un acide ribonucléique (ARN) de synthèse ou naturel qui consiste à fragmenter l'ARN et à marquer au niveau du phosphate terminal. Ce document décrit un certain nombre de fonctions pouvant être utilisées pour le marquage en liaison avec la fragmentation comme les fonctions hydroxyle, amine, hydrazine, alcoxyamine, halogénure d'alkyle, halogénure d'alkyle de type benzylique et en particulier le dérivé 5-(bromométhyl)fluorescéine. Ces fonctions permettent de marquer les acides nucléiques, mais il faut associer une étape de fragmentation pour avoir un marquage efficace car ce marquage se produit sur le phosphate libéré lors de la fragmentation. De plus, il faut ajouter un excès important de réactif de marquage par rapport à l'ARN pour obtenir un marquage efficace ce qui induit des problèmes de bruit de fond générés par le marqueur en excès. Enfin, cette méthode ne fonctionne pas efficacement sur de l'ADN double brin.

De nouveaux réactifs encore plus efficaces du point de vue du rendement de marquage sont apparus. Ceux-ci sont spécifiques au niveau de la position de marquage et en particulier affectent pas les propriétés d'hybridation des bases impliquées dans la formation de la double hélice, par l'intermédiaire des liaisons hydrogènes, qui soient utilisables à la fois pour l'ADN et l'ARN, et enfin qui permettent de marquer indifféremment des nucléotides, des oligonucléotides, des acides nucléiques naturels ou préparés par amplification enzymatique.

La Demanderesse a déjà proposé de tels nouveaux marqueurs qui répondent aux conditions précitées et qui utilisent la fonction diazométhyle comme fonction réactive pour le marquage. C'est par exemple le cas dans :
• demande de brevet WO-A-02/090319,
• la demande de brevet WO-A-02/090584,
• la demande de brevet FR 2868071 intitulée : « Réactifs de marquage, procédés de synthèse de tels réactifs et procédés de détection de molécules biologiques », ou encore
• l'article de Laayoun et al. paru dans Bioconjugate Chem. 2003, 14, 1298-1306 et intitulé : « Aryldiazomethanes for Universal Labelling of Nucleic Acids and Analysis on DNA Chips », auxquels le lecteur peut se référer afin de mieux comprendre les modes de synthèse et d'utilisation de tels constituants.
Le contenu de ces documents ci- dessus énumérés est incorporé ici pour référence.

L'état de la technique décrit également l'utilisation de support solide notamment en silice, et plus particulièrement sous forme de poudre, de gel ou de particules magnétiques, pour purifier les acides nucléiques avant ou après marquage, dans un processus aboutissant à une détection par hybridation spécifique (concernant les puces à ADN, mais aussi les plaques ELOSA ou des formes de tests rapides). La purification avant marquage permet d'améliorer considérablement le rendement de marquage, la purification post-manquage permet d'améliorer de façon déterminante le rendement d'hybridation, et le ratio Signal/bruit, nécessaire à une bonne sensibilité du test.

C'est le cas du brevet EP-B-0.389.063 qui propose un procédé d'isolation des acides nucléiques contenant un matériel biologique complexe de départ, le procédé consistant donc à mélanger le matériel de départ avec une substance chaotropique et une phase solide, la phase solide permettant la fixation d'acides nucléiques pouvant être ultérieurement nettoyés ou élués du reste des complexes ainsi créés.
Le contenu de ce brevet est également incorporé ici pour référence.

En fait, ces deux techniques sont employées de façon indépendante, les étapes de purification et de marquage s'effectuant dans des consommables différents, avec des modes opératoires également différents. La présence de deux étapes impose donc des transferts de liquides, potentiellement facteurs de contamination, de perte de matériel, et d'une façon générale, modérément automatisable.

D'autre part, le procédé combinant le marquage et la purification sur silice présente de nombreux avantages qui étaient jusqu'à présent restés insoupçonnés de l'homme du métier.

Premier avantage, le procédé selon l'invention dans un milieu réactionnel permettant à la fois la réaction de marquage et la capture, par adsorption par exemple, des acides nucléiques sur les billes magnétiques de silice n'entraîne aucune diminution de l'efficacité du marquage, marquage qui n'est donc pas affecté par la présence de la silice magnétique.

Second avantage, le procédé est comparativement beaucoup pus rapide à mettre en oeuvre qu'un précédé en deux étapes différenciées marquage et purification.

Troisème avantage de l'invention, elle permet de concentrer les acides nucléiques marqués dans un volume très faibles, de l'ordre de 0,1 à 10 µl, les acides nucléiques étant fixés sur les billes et peuvant être élués par un simple tampon d'hybridation classique sans avoir recours à un tampon d'élution. Il est donc possible de supprimer complètement cette étape de dilution, et d'améliorer la sensibilité du procédé, les acides nucléiques étant plus concentrés lors de l'hybridation.

De plus, le processus est facilement automatisable, ce qui constitue un quatrième avantage, en raison de la flexibilité de l'utilisation de billes magnétiques, et de la relative simplicité du procédé (chauffage, lavage, élution). L'utilisation de billes magnétiques permet notamment de faire varier la capacité de capture du système très facilement, par simple modification de la quantité de bille utilisée. Le procédé peut également être utilisé dans un système utilisant un flot continu, permettant de simplifier les étapes de lavage. Il n'y a alors pas de pipetage de liquide à effectuer.

Un cinquième avantage de ce procédé reside dans le fait qu'il permet le transfert des acides nucléiques sous forme solide, c'est-à-dire adsorbés aux billes de silice magnétiques, ce qui le rend facilement utilisable au sein de microcomposants, technologie qui est en plein développement actuellement

Enfin un sixième avantage parmi d'autres, qui est également liés à l'automatisation du procédé (quatrième avantage décrit) consiste en un procédé qui peut être complètement intégré dans un seul tube, de la purification de l'acide nucléique à l'hybridation sur puce, en passantr par le marquage si, bien entendu, on se trouve dans le cadre d'un procédé ne nécessitant pas d'étape d'amplification. Cela entraîne la réduction des risques de contamination, et la diminution du nombre de consommables employés.

La présente invention concerne essentiellement un procédé de marquage et de purification d'acides nucléiques d'intérêt présents dans un échantillon biologique à traiter, consistant à :
- disposer d'un unique récipient réactionnel,
- introduire dans le récipient réactionnel :
   - l'échantillon biologique,
   - au moins un réactif de marquage d'acides nucléiques,
   - au moins un support solide permettant l'adsorption desdits acides nucléiques,
   - tout ingrédient nécessaire au marquage des acides nucléiques et/ou à l'immobilisation desdits acides nucléiques sur le support,
- incuber le contenu du récipient réactionnel, et
- isoler les acides nucléiques ainsi marqués.

Selon un premier mode de réalisation de l'invention, le procédé de marquage et de purification ci-dessus se caractérise en ce que les acides nucléiques traités peuvent être constitués par des ADN et/ou ARN, simple brin et/ou double brins, synthétiques et/ou naturels.

Selon un deuxième mode de réalisation de l'invention, le procédé de marquage et de purification ci-dessus se caractérise en ce que le réactif de marquage peut permettre :
- la fragmentation des acides nucléiques de manière non spécifique pour générer une pluralité de fragments d'acides nucléiques, et
- à marquer une pluralité de ces fragments au niveau du phosphate terminal situé à l'extrémité 3' et /ou 5', ledit phosphate terminal ayant été libéré lors de la fragmentation.

Toujours selon ce deuxième mode de réalisation de l'invention, le marquage de l'extrémité 3' ou 5' d'un fragment d'acide nucléique peut s'effectuer par fixation, sur le phosphate en position 2', en position 3' ou en position 2'-3'-monophosphate cyclique, par rapport au ribose, d'une fonction réactive portée par un marqueur.

Encore selon ce deuxième mode de réalisation de l'invention, la fragmentation et/ou le marquage de l'extrémité 3' ou 5' d'un fragment d'acide nucléique peut s'effectuer par fixation, sur le phosphate en position 2', en position 3' ou en position 2'-3'-monophosphate cyclique, par rapport au ribose, d'une fonction nucléophile, électrophile, halogénure portée par un marqueur.

Selon les variantes de réalisation de ce deuxième mode de réalisation de l'invention, la fragmentation des acides nucléiques peut s'effectuer par voie :
- enzymatique (nucléases),
- chimique (cations métalliques, tels qu'ions Mg⁺⁺, Mn⁺⁺, Cu⁺⁺, Co⁺⁺ et/ou Zn⁺⁺, associés ou non à un catalyseur chimique, par exemple le N-méthyl-imidazole, ou toute molécule chimique ayant une affinité pour l'ARN et portant un noyau imidazole ou un analogue substitué) ou
- physique (par sonication ou par radiation).

Quel que soit le mode réalisation, le marquage de l'extrémité 3' ou 5' d'un fragment d'ARN peut s'effectuer par fixation, sur le phosphate relié à la position 2', à la position 3' ou à la position 2'-3'-monophosphate cyclique du ribose, d'une molécule R-X, où R est constitué par le marqueur et X est l'agent de liaison entre le marqueur et l'ARN, tel qu'un groupement hydroxyle, amine, hydrazine, alcoxylamine, halogénure d'alkyle, phényl-méthyle halogénure, iodoacétamide, maléimide.

Quel que soit le mode réalisation, le marquage sur le groupe phosphate peut être réalisé par l'intermédiaire de la 5-(bromométhyl)-fluorescéine.

Selon un mode particulier de réalisation, le procédé de marquage et de purification, selon l'invention, se caractérise en ce que le réactif de marquage peut être mis au contact des acides nucléiques en solution homogène, dans un tampon sensiblement aqueux, ledit réactif de marquage étant stable à la température et de formule (0) : 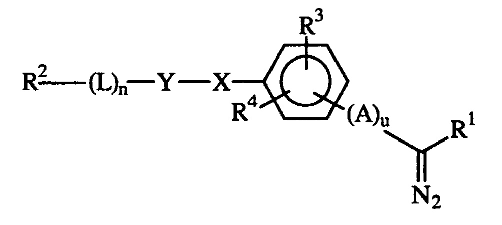 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle,
• A est un bras de liaison comportant au moins une double liaison covalente permettant la conjugaison de la fonction diazo avec le cycle aromatique et u est un nombre entier compris entre 0 et 2, préférentiellement de 0 ou 1, et
• -Y-X- représente -CONH-, -NHCO-, -CH₂O-, -CH₂S-.

Selon ce mode particulier de réalisation, le réactif de marquage peut être de formule (1) : 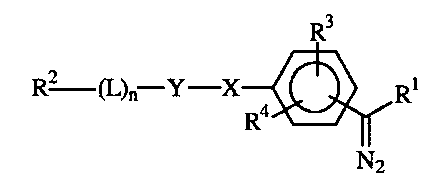 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle, et
• -Y-X- représente -CONH-, -NHCO-, -CH₂O-, -CH₂S-.
   Selon les deux précédentes caractéristiques, le réactif peut être de formule (2) : 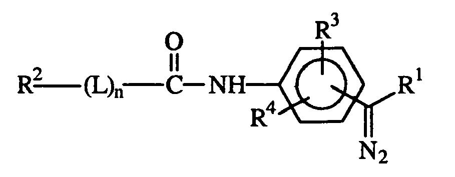
dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Selon une première variante des deux précédents modes de réalisation, le réactif peut être de formule (3) : 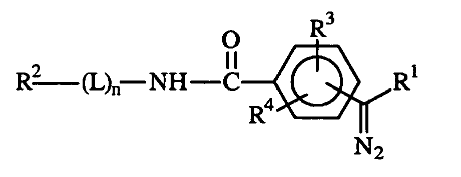 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Selon une deuxième variante des deux précédents modes de réalisation, le réactif peut être de formule (4) : 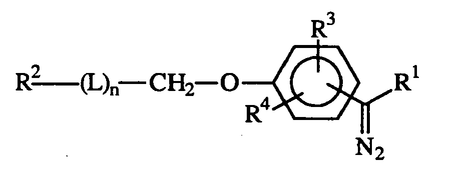 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Selon une autre variante du premier de ces deux précédents modes de réalisation, le réactif peut être de formule (21) : 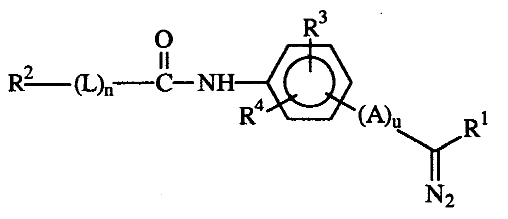 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Selon encore une autre variante du premier de ces deux précédents modes de réalisation, le réactif peut être de formule (22) : 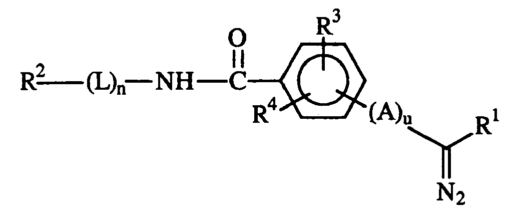 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

Toujours selon une autre variante du premier de ces deux précédents modes de réalisation, le réactif peut être de formule (23) : 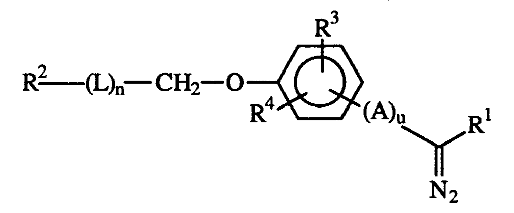 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• R³ et R⁴ représentent indépendamment l'un de l'autre : H, NO₂, Cl, Br, F, I, R² -(L)ₙ- Y-X-, OR, SR, NR₂, R, NHCOR, CONHR, COOR avec R = alkyle ou aryle.

Selon toutes les variantes de ces deux précédents modes de réalisation, le réactif, R³ et R⁴ peuvent représenter indépendamment l'un de l'autre : H, NO₂, OCH₃, -CO-NH-(CH₂)₃-(O-CH₂₋CH ₂)₃-CH₂-NH-R², -CO-NH-(CH₂)₃-(O-CH₂-CH₂)₄-CH₂-NH-R².

Selon une autre variante de ces précédents modes de réalisation, le réactif peut être de formule (7) : 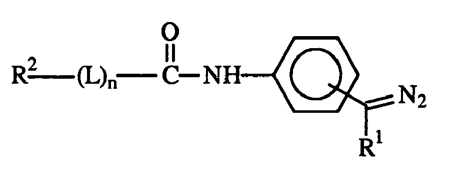 dans laquelle :
• R¹ représente H ou un groupe alkyle, aryle ou aryle substitué,
• R² représente un marqueur détectable ou au moins deux marqueurs détectables reliés entre eux par au moins une structure multimérique, et
• L est un bras de liaison comportant un enchaînement linéaire d'au moins deux liaisons covalentes et n un nombre entier égal à 0 ou 1.

Selon encore une autre variante de ces précédents modes de réalisation, le réactif peut être de formule (24) : 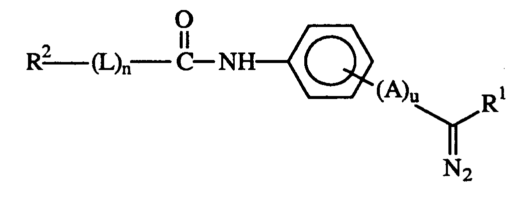 dans laquelle :
• R¹ représente H ou un groupe alkyle, aryle ou aryle substitué,
• R² représente un marqueur détectable ou au moins deux marqueurs détectables reliés entre eux par au moins une structure multimérique, et
• A est un bras de liaison comportant au moins une double liaison covalente permettant la conjugaison de la fonction diazo avec le cycle aromatique et u égal 1.

Quelle que soit la variante de ces précédents modes de réalisation, la structure R² -(L)ₙ- du réactif peut être de formule (5) : 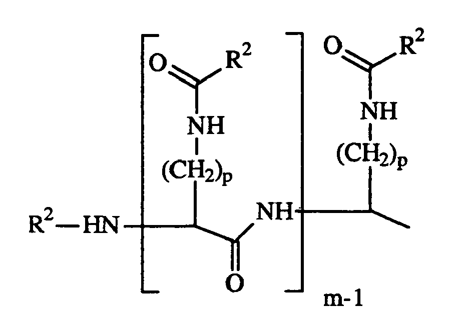 dans laquelle :
• R² représente un marqueur détectable,
• m est un nombre entier compris entre 1 et 100, et
• p est un nombre entier compris entre 1 et 10.

Quelle que soit la variante de ces précédents modes de réalisation, le réactif peut être de formule (6) : 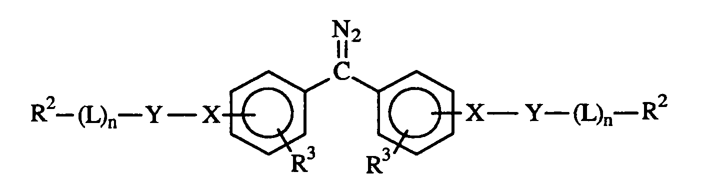 dans laquelle :
• R² représente un marqueur détectable ou au moins deux marqueurs détectables reliés entre eux par au moins une structure multimérique,
• R³ représente H, NO₂, Cl, Br, F, I, R² -(L)ₙ-Y-X-, OR, SR, NR₂, R, NHCOR, CONHR, COOR avec R = alkyle ou aryle,
• L est un bras de liaison comportant un enchaînement linéaire d'au moins deux liaisons covalentes et n un nombre entier égal à 0 ou 1, et
• -Y-X- représente -CONH-, -NHCO-, -CH₂O- -CH₂S-.

Quelle que soit la variante de ces précédents modes de réalisation, le réactif peut être de formule (25) : 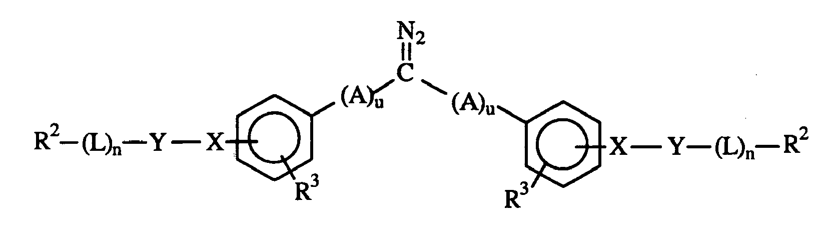 dans laquelle :
• R² représente un marqueur détectable ou au moins deux marqueurs détectables reliés entre eux par au moins une structure multimérique,
• R³ représente H, NO₂, Cl, Br, F, I, R² -(L)ₙ-Y-X-, OR, SR, NR₂, R, NHCOR, CONHR, COOR avec R = alkyle ou aryle,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• -Y-X- représente -CONH-, -NHCO-, -CH₂O-, -CH₂S-.

Quelle que soit la variante de ces précédents modes de réalisation, le réactif peut être de formule (14) : 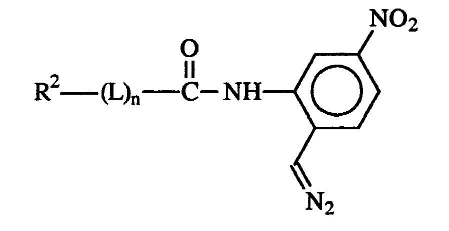 dans laquelle :
• R² représente un marqueur détectable ou au moins deux marqueurs détectables reliés entre eux par au moins une structure multimérique, et
• L est un bras de liaison comportant un enchaînement linéaire d'au moins deux liaisons covalentes et n un nombre entier égal à 0 ou 1.

Quelle que soit la variante de ces précédents modes de réalisation, le réactif peut être de formule (26) : 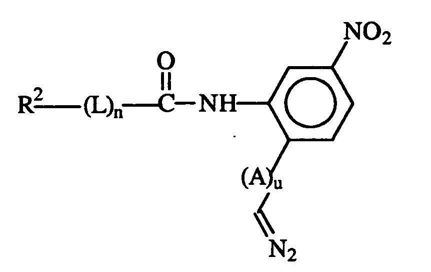 dans laquelle:
• R² représente un marqueur détectable ou au moins deux marqueurs détectables reliés entre eux par au moins une structure multimérique,
• A est un bras de liaison comportant au moins une double liaison covalente permettant la conjugaison de la fonction diazo avec le cycle aromatique et u égal 1, et
• L est un bras de liaison comportant un enchaînement linéaire d'au moins deux liaisons covalentes et n un nombre entier égal à 0 ou 1.

Quelle que soit la variante de ces précédents modes de réalisation, le réactif peut être de formule (15) : 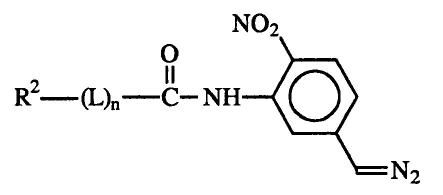 dans laquelle :
• R² représente un marqueur détectable ou au moins deux marqueurs détectables reliés entre eux par au moins une structure multimérique, et
• L est un bras de liaison comportant un enchaînement linéaire d'au moins deux liaisons covalentes et n un nombre entier égal à 0 ou 1.

Quelle que soit la variante de ces précédents modes de réalisation, le réactif peut être de formule (27): 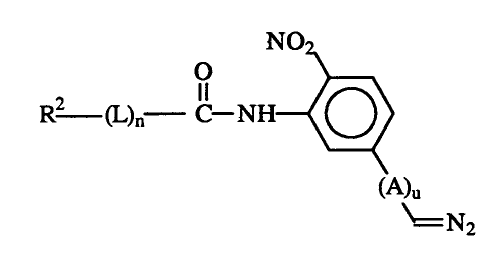 dans laquelle :
• R² représente un marqueur détectable ou au moins deux marqueurs détectables reliés entre eux par au moins une structure multimérique,
• A est un bras de liaison comportant au moins une double liaison covalente permettant la conjugaison de la fonction diazo avec le cycle aromatique et u égal 1, et
• L est un bras de liaison comportant un enchaînement linéaire d'au moins deux liaisons covalentes et n un nombre entier égal à 0 ou 1.

Selon l'ensemble des variantes de ces précédents modes de réalisation, le constituant L du réactif peut comprendre un motif -(O-CH₂-CH₂)-, répété de 1 à 20 fois, préférentiellement de 1 à 10 fois, et encore plus préférentiellement de 2 à 5 fois.

Selon les deux tout premiers modes de réalisation, le réactif de marquage peut permettre le marquage et la fragmentation d'un acide nucléique simple ou double brin selon les étapes suivantes :
- fragmenter l'acide nucléique,
- attacher un marqueur sur au moins un des fragments par l'intermédiaire d'un réactif de marquage choisi parmi les composés de formule (19) : 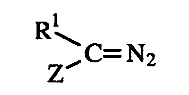
dans laquelle :
• R¹ représente H ou un groupe alkyle, aryle ou aryle substitué, et
• Z comprend un marqueur détectable,
ledit réactif se couplant de manière covalente et majoritaire sur au moins un phosphate dudit fragment.

Selon le mode de réalisation précédent, le réactif de marquage peut être choisi parmi les composés de formule (20) : 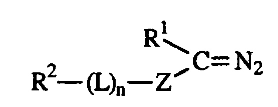 dans laquelle :
• R¹ représente H ou un groupe alkyle, aryle ou aryle substitué,
• R² est un marqueur détectable ou au moins deux marqueurs détectables reliés entre eux par au moins une structure multimérique,
• L est un bras de liaison comportant un enchaînement linéaire d'au moins deux liaisons covalentes et n est égal à 0 ou 1, et
• est choisi parmi : 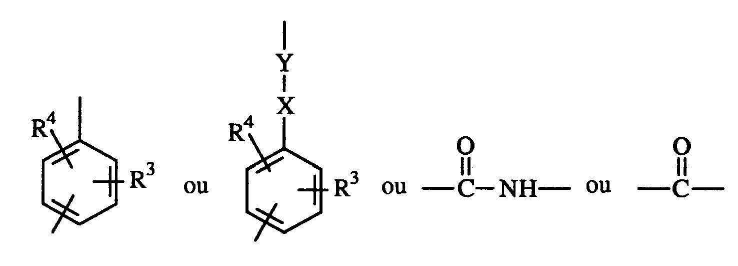
dans lequel :
■ R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle, et
■ -Y-X- représente -CONH-, -NHCO-, -CH₂O-, -CH₂S-.

Selon une variante de réalisation, groupement Z peut être constitué :

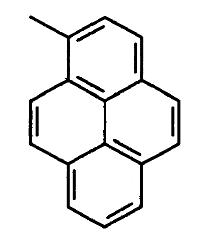

Selon une autre variante de réalisation, la fragmentation et le marquage peuvent être effectués en deux étapes.

Selon une autre variante de réalisation, la fragmentation et le marquage peuvent être effectués en une étape.

Selon une autre variante de réalisation, le marquage prut s'effectuer en solution homogène sensiblement aqueuse.

Selon une autre variante de réalisation, la fragmentation peut s'effectuer par voie enzymatique, physique ou chimique.

Selon les deux tout premiers modes de réalisation, le réactif de marquage peut être mis en contact en solution homogène, dans un tampon sensiblement aqueux, ledit réactif de marquage étant stable à la température et de formule (8) : 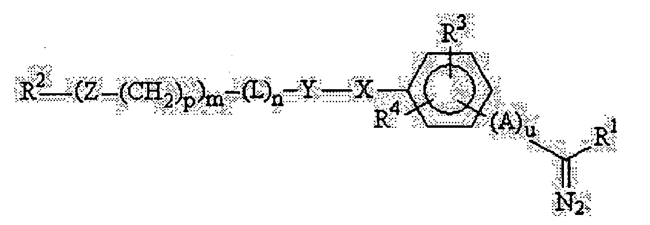 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², - CO-NH-(CH₂)₃-(O-CH₂CH₂)₄CH₂-NH-R² avec R = alkyle ou aryle,
• A est un bras de liaison comportant au moins une double liaison covalente permettant la conjugaison de la fonction diazo avec le cycle aromatique et u est un nombre entier compris entre 0 et 2, préférentiellement de 0 ou 1,
• -Y-X- représente -CONH-, -NHCO-, -CH₂O-, -CH₂S-,
• -Z- représente -NH-, -NHCO-, -CONH- ou -O-,
• m est un nombre entier compris entre 1 et 10, préférentiellement entre 1 et 3, et
• p est un nombre entier compris entre 1 et 10, préférentiellement entre 1 et 3.

Selon une variante du précédent mode de réalisation, le réactif peut être de formule (9) : 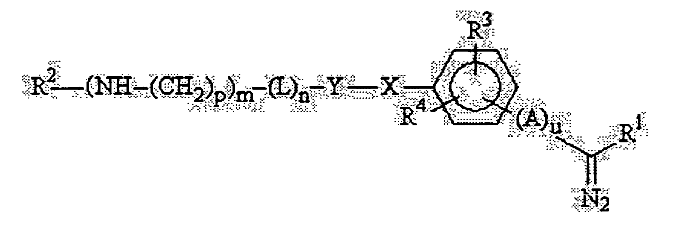 dans laquelle :
**•** R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR, -CO-NH-(CH₂)₃-(O-CH₂CH₂)₃-CH₂-NH-R², - CO-NH- (CH₂)₃- (O-CH₂-CH₂)₄-CH₂-NH-R² avec R = alkyle ou aryle, et
• -Y-X- représente -CONH-, -NHCO-, -CH₂O-, -CH₂S-,
• m est un nombre entier compris entre 1 et 10, préférentiellement entre 1 et 3, et
• p est un nombre entier compris entre 1 et 10, préférentiellement entre 1 et 3.

Selon les deux précédents modes de réalisation, dans la formule du réactif, p peut être inférieur ou égal à m

Selon les trois précédents modes de réalisation, le réactif peut être de formule (10) : 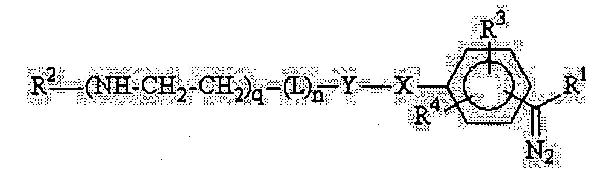 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², - CO-NH-(CH₂)₃-(O-CH₂-CH₂)₄-CH₂-NH-R² avec R = alkyle ou aryle, et
• q est un nombre entier compris entre 1 et 10, préférentiellement entre 1 et 3

Selon les quatre précédents modes de réalisation, le réactif peut être de formule (11) : 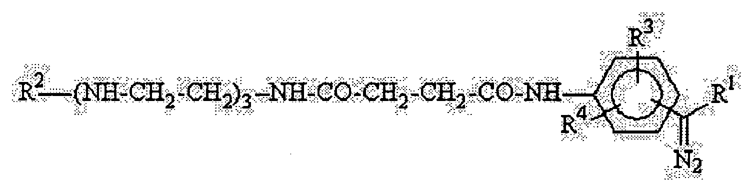 dans laquelle :
R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², - CO-NH-(CH₂)₃-(O-CH₂-CH₂)₄-CH₂-NH-R² avec R = alkyle ou aryle.

Selon un mode de réalisation, le réactif, R² peut être consituté par un résidu D-Biotine de formule (12):

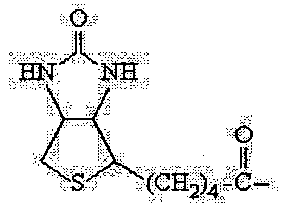

Selon les six précédents modes de réalisation, le réactif, R¹ est consituté de : CH₃, et R³ et R⁴ représentent chacun : H.

Selon les sept précédents modes de réalisation, la structure -(L)ₙ- du réactif peut être constituée par :
• la spermine ou N,N-Bis(3-aminopropyl)-1,4-diaminobutane : NH₂-(CH₂)₃-NH-(CH₂)₄-NH-(CH₂)₃-NH₂, ou
• la spermidine ou N-(3-ammopropyl)-1,4-butandiamine : H₂N-(CH₂)₄-NH-(CH₂)₃-NH₂, ou
• un dérivé contenant un motif alanine : NH₂,-CH₂-CH₂-COOH.

Selon les deux tout premiers modes de réalisation, le réactif de marquage stable à la température peut être mis en contact en solution homogène, dans un tampon aqueux, et est de formule (13) : 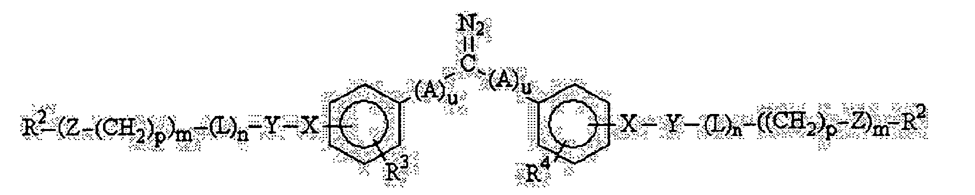 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², - CO-NH-(CH₂)₃-(O-CH₂-CH₂)₄-CH₂-NH-R² avec R = alkyle ou aryle,
• A est un bras de liaison comportant au moins une double liaison covalente permettant la conjugaison de la fonction diazo avec le cycle aromatique et u est un nombre entier compris entre 0 et 2, préférentiellement de 0 ou 1,
• -Y-X- représente -CONH-, -NHCO-, -CH₂O-, -CH₂S-,
• -Z- représente -NH-, -NHCO-, -CONH- ou -O-,
• m est un nombre entier compris entre 1 et 10, préférentiellement entre 1 et 3, et
• p est un nombre entier compris entre 1 et 10, préférentiellement entre 1 et 3. Selon le précédent mode de réalisation, le réactif peut être de formule (16) : 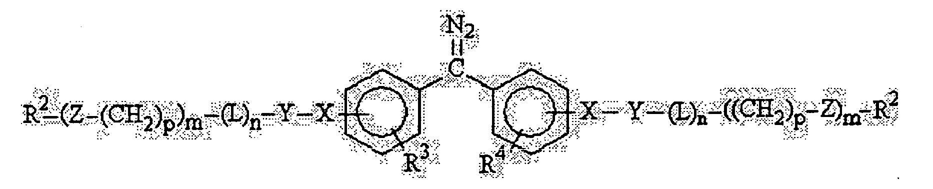
dans laquelle:
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
R³ et R⁴ représentent indépendamment l'un de l'autre : H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², - CO-NH-(CH₂)₃-(O-CH₂-CH₂)₄-CH₂-NH-R² avec R = alkyle ou aryle,
• -Y-X- représente -CONH-, -NHCO-, -CH₂O- -CH₂S-,
• -Z- représente -NH-, -NHCO-, -CONH- ou -O-,
• m est un nombre entier compris entre 1 et 10, préférentiellement entre 1 et 3, et
• p est un nombre entier compris entre 1 et 10, préférentiellement entre 1 et 3.

Selon les dix précédents modes de réalisation, le constituant L du réactif peut comprendre un motif -(O-CH₂-CH₂)-, répété de 1 à 20 fois, préférentiellement de 1 à 10 fois, et encore plus préférentiellement de 2 à 5 fois, -Z- étant alors représenté par -NH-, -NHCO- ou -CONH-.

Quel que soit le mode de réalisation, le support solide peut être constitué par des particules de silice.

Quel que soit le mode de réalisation, le support solide peut être constitué par des particules magnétiques recouvertes de silice.

Selon les deux précédents modes de réalisation, les particules de silice constituant le support solide peuvent avoir des dimensions de particules comprises entre 0,1 et 500 µm et préférentiellement entre 1 et 200 µm.

Quel que soit le mode de réalisation, un des ingrédients supplémentaires permettant le marquage peut être consitué par un alcool de préférence de l'Isopropanol.

Selon le précédent mode de réalisation, l'isopropanol peut constituer 70 % v/v du mélange final.

Quel que soit le mode de réalisation, un des ingrédients supplémentaires permettant la libération cellulaire et donc l'adsorption des acides nucléiques sur le support solide peut être consitué par un agent chaotropique.

Selon le précédent mode de réalisation, l'agent chaotropique employé peut être un sel de guanidium, de l'iodure de sodium, de l'iodure de potatium, l'(iso)thiocyanate de sodium, l'urée ou des mélanges de ces dérivés.

Selon le précédent mode de réalisation, le sel de guanidium employé peut être l'(iso)thiocyanate de guanidium.

Quel que soit le mode de réalisation, on peut séparer les complexes phase solide-acide nucléique des liquides par sédimention et rejet du surnageant et ensuite lavage des complexes par un tampon de lavage contenant une substance chaotropique.

Selon le précédent mode de réalisation, les complexes phase solide-acide nucléique, lavés par le tampon de lavage, peuvent être encore lavés ensuite à l'aide d'un ou de plusieurs solvants organiques, et ensuite soumis à un séchage.

Selon le précédent mode de réalisation, l'acide nucléique présent dans les complexes phase solide-acide nucléique, les complexes étant lavés et séchés, peut être élué au moyen d'un tampon d'élution.

Quel que soit le mode de réalisation, les complexes phase solide-acide nucléique ainsi obtenus peuvent être mis au contact avec un mélange dans lequel les composants sont présents en vue d'amplifier l'acide nucléique, soit fixé à ladite phase solide, soit élué de celle-ci.

Quel que soit le mode de réalisation, l'étape d'incubation peut consister à maintenir l'échantillon traité pendant 5 à 45 minutes, préférentiellement pendant 15 à 35 minutes, et encore plus préférentiellement pendant 25 minutes à une température comprise entre 45 et 85 °C, préférentiellement entre 55 à 75°C, et encore plus préférentiellement à 65°C.

Selon le précédent mode de réalisation, après l'étape d'incubation, l'échantillon peut être ramené à la température ambiante pendant au moins quelques minutes, préférentiellement 5 minutes.

Par « structure multimérique », on entend un polymère formé d'unités répétées de synthons chimiques ou biologiques. Un exemple est cité dans l'exemple 34.2 de la description de la demande de brevet WO-A-02/090319. L'homme du métier est invité à se référer à ce document si celui-ci trouvait les informations ci-après développées insuffisantes pour sa complète compréhension sur ce sujet. De nombreuses variantes de telles structures utilisables dans la présente invention sont connues, comme par exemple :
• les polymères linéaires (EP-A 0.561.722, EP-A-0.669.991),
• les polymères ramifiés (WO-A-01/92361),
• les particules (EP-A-0 827 552),
• les dendrimères (US-A-4,507,466 ; US-A-4,568,737 ; US-A-6,083,708),
• les polynucléotides, et
• les polypeptides.
Si cela s'avérait nécessaire, l'homme du métier peut également se référer à ces documents pour une parfaite compréhension sur ce sujet.

Par « marqueur détectable », on entend au moins un marqueur capable de générer directement un signal détectable. Par exemple la présence de biotine est considéré comme un marquage direct, car elle est détectable, même s'il est possible de l'associer ultérieurement avec de la streptavidine marquée. Une liste non limitative de ces marqueurs suit :
• les enzymes qui produisent un signal détectable par exemple par colorimétrie, fluorescence, luminescence, comme la péroxydase de raifort, la phosphatase alcaline, la β-galactosidase, la glucose-6-phosphate déshydrogénase,
• les chromophores comme les composés fluorescents, luminescents, colorants,
• les groupements à densité électronique détectable par microscopie électronique ou par leur propriété électrique comme la conductivité, l'ampérométrie, la voltamétrie, l'impédance,
• les groupements détectables, par exemple dont les molécules sont de tailles suffisantes pour induire des modifications détectables de leurs caractéristiques physiques et/ou chimiques, cette détection peut être réalisée par des méthodes optiques comme la diffraction, la résonance plasmon de surface, la variation de surface, la variation d'angle de contact ou des méthodes physiques comme la spectroscopie de force atomique, l'effet tunnel,
• les molécules radioactives comme le ³²P, le ³⁵S ou le ¹²⁵I.
De préférence, le marqueur n'est pas un marqueur radioactif pour éviter les problèmes de sécurité liés à ces marqueurs.

Dans un mode de réalisation particulier de la présente invention le marqueur est détectable électrochimiquement, et en particulier le marqueur est un dérivé d'un complexe de fer, comme un ferrocène.

Le terme « acide nucléique » signifie un enchaînement d'au moins deux désoxyribonucléotides ou ribonucléotides comprenant éventuellement au moins un nucléotide modifié, par exemple au moins un nucléotide comportant une base modifiée, telle que l'inosine, la méthyl-5-désoxycytidine, la diméthylamino-5-désoxyuridine, la désoxyuridine, la diamino-2,6-purine, la bromo-5-désoxyuridine ou toute autre base modifiée permettant hybridation. Ce polynucléotide peut aussi être modifié au niveau de la liaison internucléotidique comme par exemple les phosphorothioates, les H-phosphonates, les alkyl-phosphonates, au niveau du squelette comme par exemple les alpha-oligonucléotides (FR 2 607 507) ou les PNA (M. Egholm et al., J. Am. Chem. Soc., 114, 1895-1897, 1992 ou les 2' O-alkyl ribose et les LNA (BW, Sun et al., Biochemistry, 4160-4169,43, 2004). L'acide nucléique peut être naturel ou synthétique, un oligonucléotide, un polynucléotide, un fragment d'acide nucléique, un ARN ribosomique, un ARN messager, un ARN de transfert, un acide nucléique obtenu par une technique d'amplification enzymatique telle que :
• PCR (Polymerase Chain Reaction), décrite dans les brevets US-A-4,683,195, US-A-4,683,202 et US-A-4,800,159, et sa dérivée RT-PCR (Reverse Transcription PCR), notamment dans un format en une étape, tel que décrit dans le brevet EP-B-0.569.272,
• LCR (Ligase Chain Reaction), exposée par exemple dans la demande de brevet EP-A-0.201.184,
• RCR (Repair Chain Reaction), décrite dans la demande de brevet WO-A-90/01069,
• 3SR (Self Sustained Sequence Replication) avec la demande de brevet WO-A-90/06995,
• NASBA (Nucleic Acid Sequence-Based Amplification) avec la demande de brevet WO-A-91/02818, et
• TMA (Transcription Mediated Amplification) avec le brevet US-A-5,399,491.
• RCA (Rolling Circle Amplification (US-6,576,448).
On parle alors d'amplicons pour désigner les acides nucléiques générés par une technique d'amplification enzymatique.

Chacune de ces modifications peut être prise en combinaison pour peu qu'au moins un phosphate soit présent dans l'acide nucléique.

Par « polypeptide », on entend un enchaînement d'au moins deux acides aminés.

Par « acides aminés », on entend :
• les acides aminés primaires qui codent pour les protéines,
• les acides aminés dérivés après action enzymatique, comme la trans-4-hydroxyproline,
• les acides aminés naturels, mais non présents dans les protéines comme la norvaline, la N-méthyl-L leucine, la staline (voir Hunt S. dans Chemistry and Biochemistry of the amino acids, Barett G.C., ed., Chapman and Hall, London, 1985), et
• les acides aminés protégés par des fonctions chimiques utilisables en synthèse sur support solide ou en phase liquide et les acides aminés non naturels.

Par « étape de purification », on entend notamment la séparation entre les acides nucléiques des micro-organismes et les constituants cellulaires relargués dans l'étape de lyse qui précéde la purification des acides nucléiques. Ces étapes de lyse sont bien connues à titre d'exemple indicatif, on peut utiliser les méthodes de lyse telles que décrite dans les demandes de brevet :
- WO-A-00/60049 sur la lyse par sonication,
- WO-A-00/05338 sur la lyse mixte magnétique et mécanique,
- WO-A-99/53304 sur la lyse électrique, et
- WO-A-99/15621 sur la lyse mécanique.
L'homme du métier pourra utiliser d'autres méthodes de lyse bien connues telles que les chocs thermiques ou osmotiques ou les traitements par des agents chaotropiques, tels que les sels de guanidium (brevet US-A-5,234,809).

Cette étape permet généralement de concentrer les acides nucléiques. A titre d'exemple, on peut utiliser des particules magnétiques (voir à ce sujet les brevets US-A-4,672,040 et US-A-5,750,338), et ainsi purifier les acides nucléiques, qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet WO-A-97/45202 et WO-A-99/35500.

Le terme "support solide" tel qu'utilisé ici inclut tous les matériaux sur lesquels peut être fixé un acide nucléique. Des matériaux de synthèse ou des matériaux naturels, éventuellement modifiés chimiquement, peuvent être utilisés comme support solide, notamment les polysaccharides, tels que les matériaux à base de cellulose, par exemple du papier, des dérivés de cellulose tels que l'acétate de cellulose et la nitrocellulose, ou le dextran ; des polymères, des copolymères, notamment à base de monomères du type styrène, des fibres naturelles telles que le coton, et des fibres synthétiques telles que le nylon ; des matériaux minéraux tels que la silice, le quartz, des verres, des céramique ; des latex ; des particules magnétiques ; des dérivés métalliques, des gels, etc. Le support solide peut être sous la forme d'une plaque de microtitration, d'une membrane, d'une particule ou d'une plaque sensiblement plane de verre ou silicium ou dérivés.

Les exemples ci-joints représentent des modes particuliers de réalisation et ne peuvent pas être considérés comme limitant la portée de la présente invention.

### Exemple 1: Marquage et clivage sur phase solide du produit d'amplification PCR suivie d'une analyse sur puce Affymetrix :

### A - PCR Myco 16 S :

Cette expérience est réalisée sur un modèle dit « Myco 16S ».

Ce nom désigne les amplicons générés par PCR à partir d'une séquence de 180 bases d'un fragment du gène codant pour l'ARN ribosomal 16S de *Mycobacterium tuberculosis.*

Les conditions pour la culture, l'extraction des mycobactéries ainsi que les amorces d'amplification sont données dans la publication d'Alain Troesch. : « Mycobacterium species identification and rifampin résistance testing with high-density DNA probe arrays » paru dans J. Clin. Microbiol. 1999, 37, pages 49 à 55.

La PCR est effectuée en utilisant comme matrice de départ des préparations dADN génomique (10³ copies par PCR) avec le kit FastStart High Fidelity PCR System (Roche Diagnostic Corporation, Basel, Suisse, Référence : 03 553 426 001) avec 0,2 mM de chaque désoxyribonucléotide, 0,3 µM d'amorces et 0,4 µL d'enzyme.

Les paramètres du cycle de la PCR sont les suivants : 95°C pendant 4 minutes puis 35 cycles selon le protocole suivant : 95°C pendant 30 secondes, puis 55°C pendant 30 secondes et enfin 72°C pendant 30 secondes. Puis on conserve à 4°C jusqu'à l'arrêt du système.

Les amplicons issus de la PCR decrite ci-dessus sont désignés par la suite par les termes « PCR 16S ».

### B - Marquage en phase homogène (Contrôle) :

Le marquage en phase homogène sera utilisé comme technique de référence par rapport à l'invention, on l'appelera le « contrôle ».

Un volume de 50 µL de PCR 16S diluée au 1/5 dans du tampon d'amplification fournit dans le kit, est mélangé à 75 µL de *méta*-Biotine-PhénylMéthylDiAzoMéthane, ci-après référencé «m-BioPMDAM », (à 50 mM de Di-Méthyl SulfOxyde, ci-après désigné par « DMSO ») et 102,5 µL H₂O, puis incubés 25 minutes à 95°C. On rajoute ensuite 22,5 µL d'HCl à 0,1 M dans le milieu réactionnel puis on incube de nouveau à 95°C durant 5 minutes.

Le milieu réactionnel est ensuite purifié par le kit QIAQuick (QIAgen GmbH, Hilden, Allemagne. Référence : 28 306) en utilisant le protocole du fournisseur, puis il est hybridé sur puce à ADN (Affymetrix, Santa Clara, CA). La puce à ADN utilisée est conçue pour l'analyse de la région 213-415 de la séquence M20940 (Référence GenBank) de l'ADN 16S de *Mycobacterium tuberculosis.* Cette puce à ADN est décrite, avec le protocole d'hybridation correspondant, dans l'article d'A. Troesch et al. : «Mycobacterium species identification and rifampin résistance testing with high-density DNA probe arrays » paru dans J. Clin. Microbiol. 1999,37, pages 49 à 55.

### C - Marquage et clivage en phase hétérogène :

Parallèlement, la PCR est traitée suivant le protocole ci-dessous décrit.

Un volume de 10 µL de PCR (afin d'utiliser la même quantité de PCR que pour le contrôle décrit au paragraphe B) est mélangé à 25 µL de *m*-BioPMDAM (50 mM en DMSO), 5 µL HCL 0,2 M, 140 µL Isopropanol (% final = 70 % v/v), et 20 µL de billes de silice magnétiques MagPrep (Merck KGaA, Darmstadt, Allemagne. Référence : 1.01193.0001).

Ce mélange est incubé 25 minutes à 65°C puis 5 minutes à température ambiante. Le culot magnétique est ensuite lavé trois fois par 250 µL d'isopropanol à 70% (in H₂O 30% v/v), puis les acides nucléiques adsorbés sur la silice sont élués dans un mélange de 100 µL de tampon EB (Elution Buffer, QIAgen. Référence : 19086), auquel on rajoute 400 µL de tampon d'hybridation (6X SSPE (0,9M NaCl, 60 mM NaH₂PO₄, 6 mM EDTA, pH 7,4), 0,05% (v/v) Triton X 100. Ce tampon est préparé suivant le protocole décrit dans la publication de A. Troesch *et al.* décrit dans le paragraphe précédent, afin de reproduire les conditions utilisées pour le contrôle.

### D - Résultats et conclusion :

Les résultats en terme de pourcentage dhomologie, d'intensité du signal (I) et de bruit de fond (B) sont regroupés dans le tableau 1 suivant :

**Tableau 1 : Etude comparative du pourcentage d'homologie, d'intensité du signal (I) et de bruit de fond (B) d'un procédé utilisant l'invention par rapport à un contrôle ne l'utilisant pas**

| **Experience** | % d'homologie | I | B | I/B |
|---|---|---|---|---|
| **Contrôle** | 99,3 | 3710 | 377 | 9,8 |
| **Marquage et Clivage sur Phase Solide (invention)** | 99,3 | 4455 | 404 | 11,0 |

En conclusion, les résultats obtenus avec un procédé réactionnel selon l'invention sont comparables à ceux obtenus avec un procédé plus « classique » en deux étapes disjointes purification et marquage. De plus le procédé réactionnel est beaucoup plus court, environ 50% de temps en moins, dans le cas du marquage et clivage sur phase solide (soit 15 minutes au lieu d'une demi-heure).

On observe aussi que les valeurs d'intensités mesurées sont pratiquement similaires. Ceci indique que le rendement de purification est similaire suivant les deux méthodes de purification (QIAquick ou billes magnétiques).

### Exemple 2 : Marquage et clivage sur phase solide en présence molécule marqueur portant deux groupements biotine :

### A - Marquage en phase homogène (Contrôle) :

Un volume de 50 µL de PCR 16S diluée au 1/10 dans du tampon d'amplification (on effectue cette dilution afin de tester la sensibilité du test, en réduisant la concentration en amplicons) sont mélangés à 39,5 µL de *N*,*N*'-Bis(13-biotinoylamino-4,7,10-trioxatridécyl)-5-(diazométhyl)isophtalamide (ci-après référencé «bisBioPDAM») 190 mM en DMSO, 110,5 µL DMSO et 15 µL H₂O, puis incubés 25 minutes à 95°C. On rajoute ensuite 35 µL d'HCl à 0,1 M dans le milieu réactionnel, puis on incube de nouveau à 95°C durant 5 minutes.

Le milieu réactionnel est ensuite purifié par le kit QIAquick en utilisant le protocole du fournisseur, puis il est hybridé sur puce à ADN (Affymetrix, Santa Clara, CA) de la même manière que dans l'exemple 1.

### B - Marquage et clivage en Phase Hétérogène

Parallèlement, la même PCR diluée au 1/10 est traitée suivant un autre protocole. Le volume de 50 µL de PCR est mélangé à 39,5 µL de bisBioPDAM 190 mM en DMSO, 5 µL HCI 0,4 M, 175 µL Isopropanol (% final = 70 % v/v), 6,5 µL de DMSO, et un culot de 20 µL de billes de silice magnétiques MagPrep.

Ce mélange est incubé 15 minutes à 80°C puis 5 minutes à température ambiante. Le culot magnétique est ensuite lavé trois fois par 250 µL d'isopropanol à 70% (dans H₂O 30% v/v), puis les acides nucléiques adsorbés sur la silice sont élués dans un mélange de 100 µL de tampon d'élution EB (voir ci-dessus), auxquels on rajoute 400 µL de tampon d'hybridation. L'hybridation est réalisée de la même manière que dans l'exemple 1.

### C - Résultats et conclusions :

Les résultats en terme de pourcentage d'homologie, d'intensité du signal (I) et de bruit de fond (B) sont regroupées dans le tableau 2 suivant :

**Tableau 2 : Etude comparative du pourcentage d'homologie, d'intensité du signal (I) et de bruit de fond (B) d'un procédé utilisant l'invention par rapport à un contrôle ne l'utilisant pas**

| **Experience** | % d'homologie | I | B | I/B |
|---|---|---|---|---|
| **Contrôle** | 99,1 | 1051 | 232 | 4,5 |
| **Marquage et Clivage sur Phase Solide (invention)** | 99,1 | 4071 | 437 | 9,3 |

En conclusion, les résultats obtenus avec un procédé réactionnel selon l'invention sont meilleurs que ceux obtenus avec un procédé plus «classique » en deux étapes disjointes purification et marquage lorsque l'on utilise la bisBioPDAM, sur une PCR diluée.

Cette molécule, plus soluble que le *m-* BioPMDAM, portant deux groupements biotines, permet d'autre part d'améliorer la sensibilité du test utilisant le marquage sur support solide. Ceci est visible dans l'amélioration des valeurs d'intensité (I) et de rapport signal sur bruit (I/B) qui sont améliorées par rapport au procédé « classique ».

L'utilisation du procédé réactionnel et de la bisBioPDAM permet donc d'obtenir un test robuste (forte valeur d'intensité, peu sensible aux variations inter-tests) pour un nombre de copies plus faibles.

### Exemple 3: Marquage sur phase solide de produits d'amplification PCR suivie d'une analyse quantitative sur puce Agilent:

### A-PCR VHB

Le terme «PCR VHB » désigne dans la suite du texte le produit d'amplification par PCR d'un fragment de 3200 paires de bases du génome du virus de l'hépatite B, qui est généré de la manière suivante.

Approximativement 10³ copies d'ADN de virus de l'hépatite B (VHB), contenus dans un volume de 15 µL de surnageant d'une culture cellulaire, sont amplifiées par PCR en utilisant le kit Expand High Fidelity (Roche Diagnostic corporation, Basel, Suisse. Référence : 1732 641).

Pour information, le milieu réactionnel final utilisé dans le kit contient 1,5 mM de MgCl₂, 200 µM de chaque dNTP, et 2,6 Unités d'un mélange d'ADN Polymérases *Taq* et *Pow* ; 0,3 µM d'amorces P1 et P2 dont les séquences sont :
P1 : 5'-CCGGAAAGCTTGAGCTCTTCTTTTTCACCTCTGCTAATCA-3'
P2 :5'-CCGGAAAGCTTGAGCTCTTCAAAAAGTTGCATGGTGCTGG-3',
sont rajoutés au mélange, afin de permettre l'amplification du fragment de 3200 nucléotides attendu.

Une description de cette PCR est présentée dans l'article de Gunther S. et al. « A novel method for efficient amplification of whole hepatitis B virus genome permits rapid functionnal analysis and reveals deletion mutants in immunosuppressed patients », Journal of Virology, September 1995, pages 5437 à 5444.

Cette PCR VHB est utilisée afin de disposer d'une PCR agissant sur des séquences d'acides nucléiques beaucoup plus longues qu'avec le modèle myco (3200 bases au lieu de 180 pour myco 16S) pour étudier le rendement de capture des billes magnétiques sur deux modèles très différents.

### B - Détermination des rendements de capture :

Un produit d'amplification par PCR d'une partie du gène 16S de *Mycoplasma tuberculosis,* amplifié de la même manière que décrit dans l'exemple 1, et parallèlement un produit de PCR VHB sont traités de la façon suivante :

Un volume de 10 µL de PCR est mélangé à 25 µL de *m-*BioPMDAM (50 mM en DMSO), 5 µL H₂O, 140 µL Isopropanol (% final = 70 % v/v), et 20 µL de billes de silice magnétique MagPrep.

Ce mélange est incubé 25 min à 65°C puis 5 min à température ambiante. Le culot magnétique est ensuite lavé trois fois par 250 µL d'isopropanol à 70% (en H₂O 30% v/v), puis les acides nucléiques adsorbés sur la silice sont élués dans 50 µL, de tampon EB.

Parallèlement les mêmes solutions PCR sont marquées suivant le même protocole (Isopropanol remplacé par H₂O) puis purifiées suivant le protocole QIAquick et éluées dans un volume final de 50 µL.

Les produits de purification ainsi que les PCR initiales sont analysées sur BioAnalyser 2100 (Agilent, Palo Alto, CA, Etats-Unis d'Amérique, Référence : G2940CA) en utilisant le protocole du fournisseur, afin de les quantifier.

### C - Résultats et conclusion :

Les valeurs obtenues sont reproduites dans le tableau 3 reproduit ci-dessous :

**Tableau 3 : Comparaison du rendement de capture d'acides nucléiques dans le milieu réactionnel par un procédé classique de l'état de la technique et un procédé selon l'invention**

| | **Initial** | **QIAgen** | **Invention** |
|---|---|---|---|
| **VHB (ng/µL)** | 8,2 | 3,6 | 7,7 |
| **% Rendement** | | 44 | 94 |
| | | | |
| **Myco (ng/µL)** | 2,7 | 0,94 | 2,6 |
| **% Rendement** | | 35 | 96 |

Pour conclure, ces résultats ajoutés à ceux obtenus sur puce, montrent que le re-largage d'acides nucléiques dans le milieu réactionnel est faible. Dans le cadre du kit QIAgen, l'essentiel de la capture s'effectue sur des fragments de petite taille, ce qui permet son utilisation avec les produits de clivage hybridés, l'hybridation sur puce à ADN nécessitant dans certains cas un clivage des produits de PCR (voir par exemple Zhang Y. et al. « Reproducible and inexpensive probe préparation for oligonucleotide arrays », paru dans Nucleic Acids Res. 2001, 29, e66. Les résultats présentés dans cet exemple permettent de montrer que le protocole utilisé avec les billes magnétiques permet de capturer une gamme plus large de taille de fragments. Ceci permet une plus grande flexibilité de la technologie, puisqu'il est possible, à partir de produit de PCR de tailles très variées d'obtenir, par modulation du clivage, des fragments d'ADN marqués de taille adaptée à l'application envisagée, sans contrainte technique notable.

### Exemple 4: Marquage et clivage sur phase solide d'ADN génomique suivi d'une analyse sur puce Affymetrix :

### A - Préparation de l'ADN génomique :

L'ADN génomique, qui sera par la suite appelé «ADNg », est extrait à partir d'une culture liquide de *Staphylococcus aureus* de 18 heures en milieu Trypticase soja (bioMérieux, Marcy, France. Référence : 41 146).

L'ADNg est purifié par colonne d'échange d'ions « Genomic Tips 500 » (QIAgen. Référence : 10262) suivant le protocole du fournisseur, et quantifié par mesure d'absorbance à 260 nm.

### B - Marquage et hybridation de l'ADNg :

Un volume de 10 µL d'ADNg, contenant environ 10⁹ copies de génomes bactérien est mélangé à 25 µL de *m-*BioPMDAM (50 mM en DMSO), 5 µL d'acétate de sodium pH = 3, 40 µL Isopropanol (% final = 70 % v/v), et 20 µL de billes de silice magnétiques MagPrep (Merck).

Ce mélange est incubé 25 minutes à 65°C puis 5 minutes à température ambiante. Le culot magnétique est ensuite lavé trois fois par 250 µL d'isopropanol à 70% (in H₂O 30% v/v), puis les acides nucléiques adsorbés sur la silice sont élués dans un mélange de 100 µL de tampon EB auquel on rajoute 400 µL de tampon d'hybridation (Tetra Metyl Ammonium Chloride, 3 M ; Tris, pH 7,8, 10 mM ; Tween-20, 0,01% ; Acetylated Bovine Serum Albumin, 500 µg/mL ; Hering Sperm DNA, 100 µg/mL. Ce tampon est préparé suivant le protocole fournit par Affymetrix dans son manuel utilisateur : CustomSeq resequencing Array protocol Version 2.0).

Le mélange est hybridé sur une puce Affymetrix conçu pour l'analyse du gène 16S dont les caractéristiques et le protocole opératoire sont décrit dans l'article de G. Vernet et al. « Species differentiation and antibiotic susceptibility testing with DNA microarrays », paru dans J. AppL Microbiol., 2004, 96, pages 59 à 68 et C. Jay et al. « 16S rRNA gene-based identification of Staphylococcus species using high densityDNA probe array», 10th international Symposium on staphylococci and Staphylococcal infections Tsukuba, d'octobre 2002.

### C- Résultats et conclusion :

Les résultats en terme de pourcentage d'homologie, d'intensité du signal (I) et de bruit de fond (B) sont regroupées dans le tableau 4 suivant :

**Tableau 4 : Résultats de pourcentage d'homologie, d'intensité du signal (I) et de bruit de fond (B) d'un procédé de traitement de l'ADNg utilisant l'invention**

| **Experience** | % d'homologie | I | B | I/B |
|---|---|---|---|---|
| **Marquage et clivage sur phase solide (invention)** | 92,9 | 2799 | 599 | 4,7 |

En conclusion, ce résultat est très satisfaisant. Le pourcentage d"homologie de 93% permet d'identifier de manière idéale l'espèce bactérienne analysée, et de la différencier des autres espèces pouvant être identifiées sur la puce.

Sachant qu'il est possible de purifier l'ADNg sur les même billes magnétiques que celles utilisées pour la purification de l'ADN marqué, on peut réaliser l'ensemble du protocole (de la colonie bactérienne à l'ADN prêt à être hybridé) dans un seul et même tube, traité manuellement ou dans un automate.

Des résultats identiques du point de vue de l'invention peuvent être obtenus en utilisant d'autres techniques d'amplification comme la NASBA ou TMA, qui génèrent directement des amplicons ARN.

## Revendications

1. Procédé de marquage et de purification d'acides nucléiques d'intérêt présents dans un échantillon biologique à traiter, consistant à :
• disposer d'un unique récipient réactionnel,
• introduire dans le récipient réactionnel:
- l'échantillon biologique,
- au moins un réactif de marquage d'acides nucléiques, ledit réactif de marquage étant stable à la température et de formule (0) : 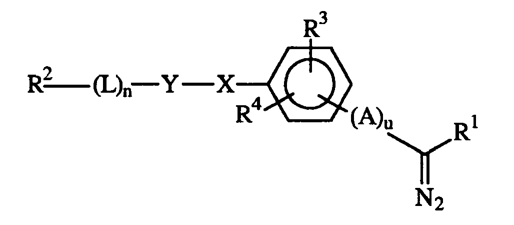 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R²-(L)ₙ-Y-X-, OR, SR, NR₂, R, NHCOR, CONHR, COOR avec R = alkyle ou aryle,
• A est un bras de liaison comportant au moins une double liaison covalente permettant la conjugaison de la fonction diazo avec le cycle aromatique et u est un nombre entier compris entre 0 et 2, préférentiellement de 0 ou 1, et
• Y-X- représente -CONH-, -NHCO-, -CH₂O -CH₂S-.
- au moins un support solide permettant l'adsorption desdits acides nucléiques,
- tout ingrédient nécessaire au marquage des acides nucléiques et/ou à l'immobilisation desdits acides nucléiques sur le support,
• incuber le contenu du récipient réactionnel, et
• isoler les acides nucléiques ainsi manqués.

2. Procédé de marquage et de purification, selon la revendication 1, **caractérisé en ce que** les acides nucléiques traités sont constitués par des ADN et/ou ARN, simple brin et/ou double brins, synthétiques et/ou naturels.

3. Procédé de marquage et de purification, selon l'une quelconque des revendications 1 ou 2, **caractérisé en ce que** l'introduction du réactif de marquage permet :
- la fragmentation des acides nucléiques de manière non spécifique pour générer une pluralité de fragments d'acides nucléiques, et
- à marquer une pluralité de ces fragments au niveau du phosphate terminal situé à l'extrémité 3' et /ou 5', ledit phosphate terminal ayant été libéré lors de la fragmentation.

4. Procédé de marquage et de purification, selon la revendication 3, **caractérisé en ce que** le marquage de l'extrémité 3' ou 5' d'un fragment d'acide nucléique s'effectue par fixation, sur le phosphate en position 2', en position 3' ou en position 2'-3'-monophosphate cyclique, par rapport au ribose, d'une fonction réactive portée par un marqueur.

5. Procédé de marquage et de purification, selon la revendication 3, **caractérisé en ce que** la fragmentation et/ou le marquage de l'extrémité 3' ou 5' d'un fragment d'acide nucléique s'effectue par fixation, sur le phosphate en position 2', en position 3' ou en position 2'-3'-monophosphate cyclique, par rapport au ribose, d'une fonction nucléophile, électrophile, halogénure portée par un marqueur

6. Procédé, selon l'une quelconque des revendications 3 à 5, **caractérisé en ce que** la fragmentation des acides nucléiques s'effectue par voie :
- enzymatique (nucléases),
- chimique (cations métalliques, tels qu'ions Mg⁺⁺, Mn⁺⁺, Cu⁺⁺, Co⁺⁺ et/ou Zn⁺⁺, associés ou non à un catalyseur chimique, par exemple le N-méthyl-imidazole, ou toute molécule chimique ayant une affinité pour l'ARN et portant un noyau imidazole ou un analogue substitué) ou
- physique (par sonication ou par radiation).

7. Procédé, selon l'une quelconque des revendications 3 à 6, **caractérisé en ce que** le marquage de l'extrémité 3' ou 5' d'un fragment d'ARN s'effectue par fixation, sur le phosphate relié à la position 2', à la position 3' ou à la position 2'-3'-monophosphate cyclique du ribose, d'une molécule R-X, où R est constitué par le marqueur et X est l'agent de liaison entre le marqueur et l'ARN, tel qu'un groupement hydroxyle, amine, hydrazine, alcoxylamine, halogénure d'alkyle, phényl-méthyle halogénure, iodoacétamide, maléimide.

8. Procédé, selon l'une quelconque des revendications 3 à 7, **caractérisé en ce que** le marquage sur le groupe phosphate est réalisé par l'intermédiaire de la 5-(bromométhyl)-fluorescéine.

9. Procédé de marquage et de purification, selon l'une quelconque des revendications 1 ou 2, **caractérisé en ce que** le réactif de marquage est mis au contact des acides nucléiques en solution homogène, dans un tampon sensiblement aqueux, ledit réactif de marquage étant stable à la température et de formule (0).

10. Procédé, selon la revendication 9, dans lequel le réactif de marquage utilisé est de formule (1) : 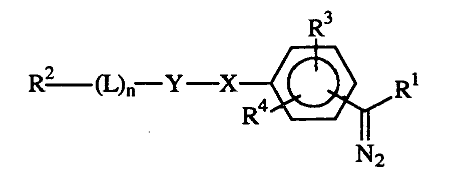 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ- Y-X-, OR, SR, NR₂, R, NHCOR, CONHR, COOR avec R = alkyle ou aryle, et
• -Y-X- représente -CONH-, -NHCO-, -CH₂O- -CH₂S-.

11. Procédé, selon l'une quelconque des revendications 9 ou 10, dans lequel le réactif est de formule (2) : 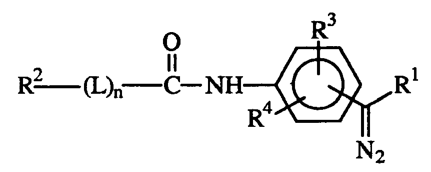 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

12. Procédé, selon l'une quelconque des revendications 9 ou 10, dans lequel le réactif est de formule (3) : 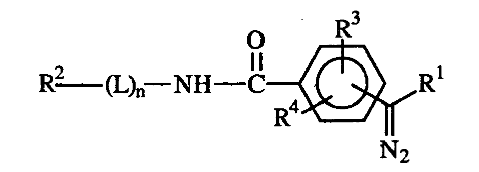 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

13. Procédé, selon l'une quelconque des revendications 9 ou 10, dans lequel le réactif est de formule (4) : 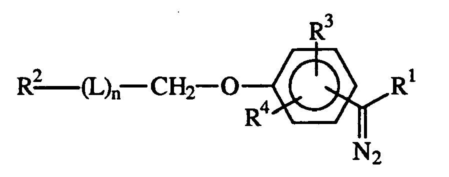 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

14. Procédé, selon la revendication 9, dans lequel le réactif est de formule (21) : 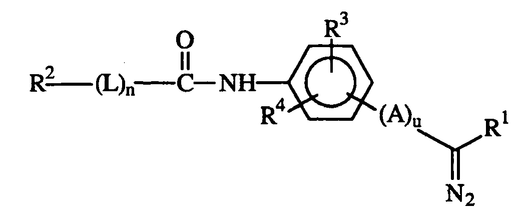 dans laquelle:
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

15. Procédé, selon la revendication 9, dans lequel le réactif est de formule (22) : 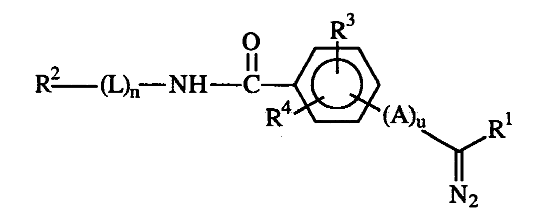 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
**•** R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

16. Procédé, selon la revendication 9, dans lequel le réactif est de formule (23) : 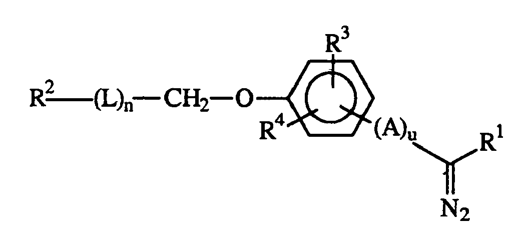 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle.

17. Procédé, selon l'une quelconque des revendications 9 à 16, **caractérisé par le fait que**, dans lequel le réactif de marquage utilisé comporte R³ et R⁴ qui représentent indépendamment l'un de l'autre : H, NO₂, OCH₃, -CO-NH-(CH₂)₃-(O-CH₂-CH₂)₃-CH₂-NH-R², -CO-NH-(CH₂)₃-(O-CH₂-CH₂)₄-CH₂-NH-R².

18. Procédé, selon l'une quelconque des revendications 9 à 11, dans lequel le réactif est de formule (7) : 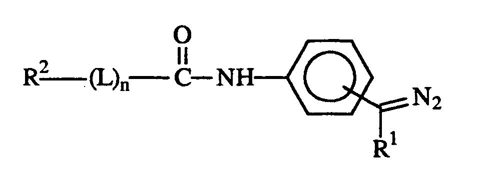 dans laquelle :
• R¹ représente H ou un groupe alkyle, aryle ou aryle substitué,
• R² représente un marqueur détectable ou au moins deux marqueurs détectables reliés entre eux par au moins une structure multimérique, et
• L est un bras de liaison comportant un enchaînement linéaire d'au moins deux liaisons covalentes et n un nombre entier égal à 0 ou 1.

19. Procédé, selon l'une quelconque des revendications 9 ou 14, dans lequel le réactif est de formule (24) : 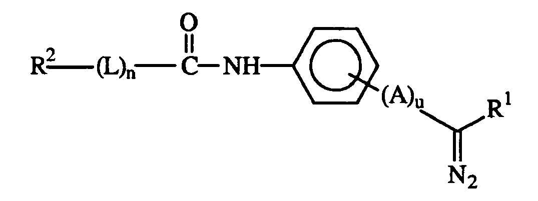 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• A est un bras de liaison comportant au moins une double liaison covalente permettant la conjugaison de la fonction diazo avec le cycle aromatique et u égal 1.

20. Procédé, selon l'une quelconque des revendications 9 à 19, dans laquelle la structure R²-(L)ₙ- du réactif est de formule (5) : 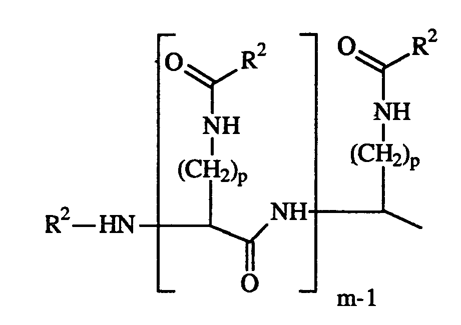 dans laquelle :
• R² représente un marqueur détectable,
• m est un nombre entier compris entre 1 et 100, et
• p est un nombre entier compris entre 1 et 10.

21. Procédé de marquage et de purification d'acides nucléiques d'intérêt présents dans un échantillon biologique à traiter, consistant à :
• disposer d'un unique récipient réactionnel,
• introduire dans le récipient réactionnel :
- l'échantillon biologique,
- au moins un réactif de marquage d'acides nucléiques, le réactif de marquage étant mis au contact des acides nucléiques en solution homogène, dans un tampon sensiblement aqueux, ledit réactif de marquage étant stable à la température, et est de formule (6) : 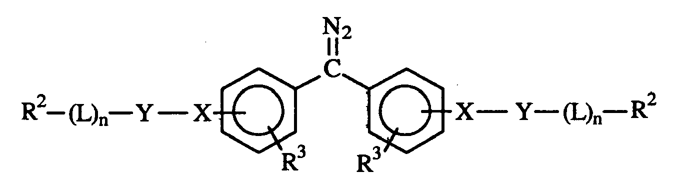 dans laquelle :
• R² représente un marqueur détectable ou au moins deux marqueurs détectables reliés entre eux par au moins une structure multimérique,
• R³ représente H, NO₂, Cl, Br, F, I, R² -(L)ₙ-Y-X-, OR, SR, NR₂, R, NHCOR, CCNHR, COOR avec R = alkyle ou aryle,
• L est un bras de liaison comportant un enchaînement linéaire d'au moins deux liaisons covalentes et n un nombre entier égal à 0 ou 1, et
• -Y-X- représente -CONH-, -NHCO-, -CH₂O-, -CH₂S-,
- au moins un support solide permettant l'adsorption desdits acides nucléiques,
- tout ingrédient nécessaire au marquage des acides nucléiques et/ou à l'immobilisation desdits acides nucléiques sur le support,
• incuber le contenu du récipient réactionnel, et
• isoler les acides nucléiques ainsi marqués.

22. Procédé de marquage et de purification d'acides nucléiques d'intérêt présents dans un échantillon biologique à traiter, consistant à :
• disposer d'un unique récipient réactionnel,
• introduire dans le récipient réactionnel:
- l'échantillon biologique,
- au moins un réactif de marquage d'acides nucléiques, le réactif de marquage étant mis au contact des acides nucléiques en solution homogène, dans un tampon sensiblement aqueux, ledit réactif de marquage étant stable à la température, et est de formule (25) : 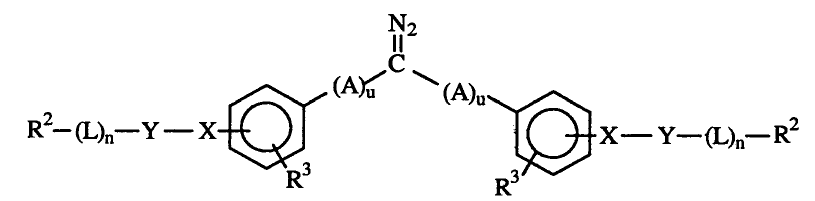 dans laquelle :
• R² représente un marqueur détectable ou au moins deux marqueurs détectables reliés entre eux par au moins une structure multimérique,
• R³ représente H, NO₂, Cl, Br, F, I, R² -(L)ₙ-Y-X-, OR, SR, NR₂, R, NHCOR, CONHR, COOR avec R = alkyle ou aryle,
• L est un bras de liaison comportant un enchaînement linéaire d'au moins deux liaisons covalentes et n un nombre entier égal à 0 ou 1,
• A est un bras de liaison comportant au moins une double liaison covalente permettant la conjugaison de la fonction diazo avec le cycle aromatique et u égal 1, et
• -Y-X- représente -CONH-, -NHCO-, -CH₂O-, -CH₂S-,
- au moins un support solide permettant l'adsorption desdits acides nucléiques,
- tout ingrédient nécessaire au marquage des acides nucléiques et/ou à l'immobilisation desdits acides nucléiques sur le support,
• incuber le contenu du récipient réactionnel, et
• isoler les acides nucléiques ainsi marqués.

23. Procédé, selon l'une quelconque des revendications 9 à 11, dans lequel le réactif est de formule (14) : 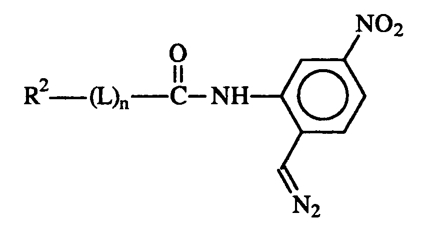 dans laquelle :
• R² représente un marqueur détectable ou au moins deux marqueurs détectables reliés entre eux par au moins une structure multimérique, et
• L est un bras de liaison comportant un enchaînement linéaire d'au moins deux liaisons covalentes et n un nombre entier égal à 0 ou 1.

24. Procédé, selon l'une quelconque des revendications 9 ou 14, dans lequel le réactif est de formule (26) : 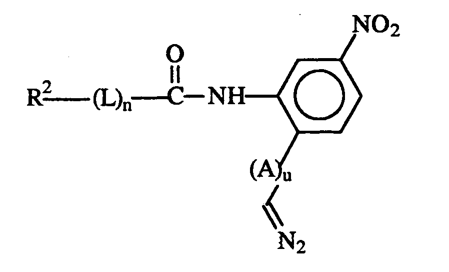 dans laquelle :
• R² représente un marqueur détectable ou au moins deux marqueurs détectables reliés entre eux par au moins une structure multimérique,
• A est un bras de liaison comportant au moins une double liaison covalente permettant la conjugaison de la fonction diazo avec le cycle aromatique et u égal 1, et
• L est un bras de liaison comportant un enchaînement linéaire d'au moins deux liaisons covalentes et n un nombre entier égal à 0 ou 1.

25. Procédé, selon l'une quelconque des revendications 9 à 11, dans lequel le réactif est de formule (15) : 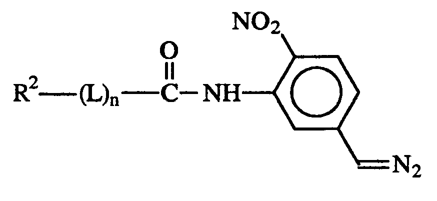 dans laquelle :
• R² représente un marqueur détectable ou au moins deux marqueurs détectables reliés entre eux par au moins une structure multimérique, et
• L est un bras de liaison comportant un enchaînement linéaire d'au moins deux liaisons covalentes et n un nombre entier égal à 0 ou 1.

26. Procédé, selon l'une quelconque des revendications 9 ou 14, dans lequel le réactif est de formule (27) : 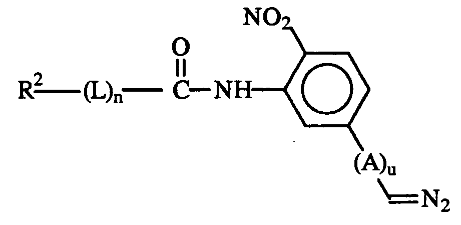 dans laquelle :
• R² représente un marqueur détectable ou au moins deux marqueurs détectables reliés entre eux par au moins une structure multimérique,
• A est un bras de liaison comportant au moins une double liaison covalente permettant la conjugaison de la fonction diazo avec le cycle aromatique et u égal 1, et
• L est un bras de liaison comportant un enchaînement linéaire d'au moins deux liaisons covalentes et n un nombre entier égal à 0 ou 1.

27. Procédé, selon l'une quelconque des revendications 9 à 26, **caractérisé par le fait que** le constituant L du réactif comprend un motif -(O-CH₂-CH₂)-, répété de 1 à 20 fois, préférentiellement de 1 à 10 fois, et encore plus préférentiellement de 2 à 5 fois.

28. Procédé de marquage et purification d'acides nucléiques d'intérêt présents dans un échantillon biologique à traiter, consistant à :
• disposer d'un unique récipient réactionnel,
• introduire dans le récipient réactionnel :
- l'échantillon biologique,
- au moins un réactif de marquage d'acides nucléiques, le réactif de marquage permettant le marquage et la fragmentation d'un acide nucléique simple ou double brin selon les étapes suivantes :
? fragmenter l'acide nucléique,
? attacher un marqueur sur au moins un des fragments par l'intermédiaire d'un réactif de marquage choisi parmi les composés de formule (19) : 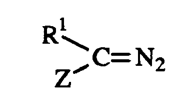 dans laquelle :
• R¹ représente H ou un groupe alkyle, aryle ou aryle substitué, et
• Z comprend un marqueur détectable,
ledit réactif se couplant de manière covalente et majoritaire sur au moins un phosphate dudit fragment,
- au moins un support solide permettant l'adsorption desdits acides nucléiques,
- tout ingrédient nécessaire au marquage des acides nucléiques et/ou à l'immobilisation desdits acides nucléiques sur le support,
• incuber le contenu du récipient réactionnel, et
• isoler les acides nucléiques ainsi marqués.

29. Procédé, selon la revendication 28, **caractérisé par le fait que** le réactif de marquage est choisi parmi les composés de formule (20) : 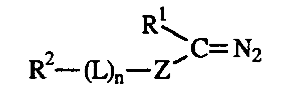 dans laquelle :
• R¹ représente H ou un groupe alkyle, aryle ou aryle substitué,
• R² est un marqueur détectable ou au moins deux marqueurs détectables reliés entre eux par au moins une structure multimérique,
• L est un bras de liaison comportant un enchaînement linéaire d'au moins deux liaisons covalentes et n est égal à 0 ou 1, et
• Z est choisi parmi : 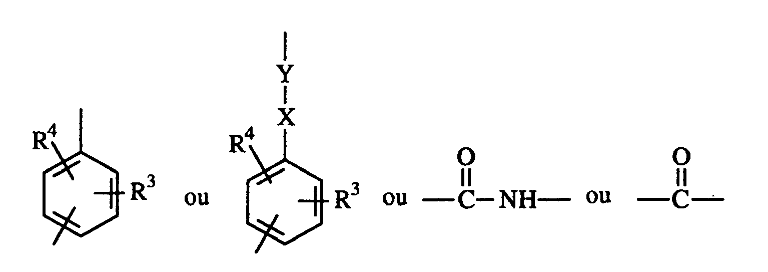 dans lequel :
■ R³ et R⁴ représentent indépendamment l'un de l'autre : H, NO₂, Cl, Br, F, I, R² -(L)ₙ-Y-X-, OR, SR, NR₂, R, NHCOR, CONHR, COOR avec R = alkyle ou aryle, et
■ -Y-X- représente -CONH-, -NHCO-, -CH₂O- -CH₂S-.

30. Procédé, selon la revendication 28, **caractérisé par le fait que** Z est :

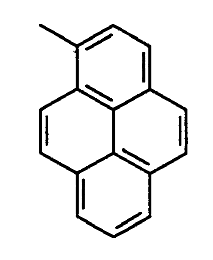

31. Procédé, selon l'une quelconque des revendications 28 à 30, **caractérisé par le fait que** la fragmentation et le marquage sont effectués en deux étapes.

32. Procédé, selon l'une quelconque des revendications 28 à 30, **caractérisé par le fait que** la fragmentation et le marquage sont effectués en une étape.

33. Procédé, selon l'un quelconque des revendications 28 à 32, **caractérisé par le fait que** le marquage s'effectue en solution homogène sensiblement aqueuse.

34. Procédé, selon l'une quelconque des revendications 28 à 33, **caractérisé par le fait que** la fragmentation s'effectue par voie enzymatique, physique ou chimique.

35. Procédé de marquage et de purification d'acides nucléiques d'intérêt présents dans un échantillon biologique à traiter, consistant à :
• disposer d'un unique récipient réactionnel,
• introduire dans le récipient réactionnel :
- l'échantillon biologique,
- au moins un réactif de marquage d'acides nucléiques, le réactif de marquage étant mis en contact en solution homogène, dans un tampon sensiblement aqueux, ledit réactif de marquage étant stable à la température et de formule (8) : 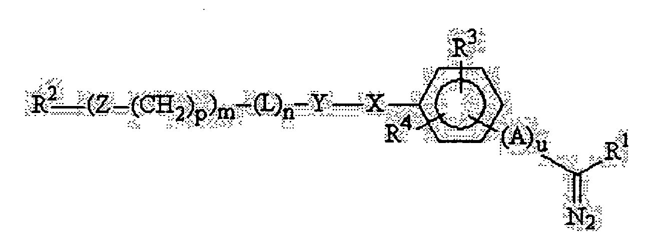 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre: H, NO₂, Cl, Br, F, I, R²-(L)ₙ-Y-X-, OR, SR, NR₂, R, NHCOR, CONHR, COOR, -CO-NH-(CH₂)₃-(O-CH₂-CH₂)₃-CH₂-NH-R², -CO-NH-(CH₂)₃-(O-CH₂-CH₂)₄-CH₂-NH-R² avec R = alkyle ou aryle,
• A est un bras de liaison comportant au moins une double liaison covalente permettant la conjugaison de la fonction diazo avec le cycle aromatique et u est un nombre entier compris entre 0 et 2, préférentiellement de 0 ou 1,
• -Y-X- représente -CONH-, -NHCO-, -CH₂O-, -CH₂S-,
• -Z- représente -NH-, -NHCO-, -CONH- ou -O-,
**•** m est un nombre entier compris entre 1 et 10, préférentiellement entre 1 et 3, et
• p est un nombre entier compris entre 1 et 10, préférentiellement entre 1 et 3,
- au moins un support solide permettant l'adsorption desdits acides nucléiques,
- tout ingrédient nécessaire au marquage des acides nucléiques et/ou à l'immobilisation desdits acides nucléiques sur le support,
• incuber le contenu du récipient réactionnel, et
• isoler les acides nucléiques ainsi marqués.

36. Procédé, selon la revendication 35, dans lequel le réactif est de formule (9) : 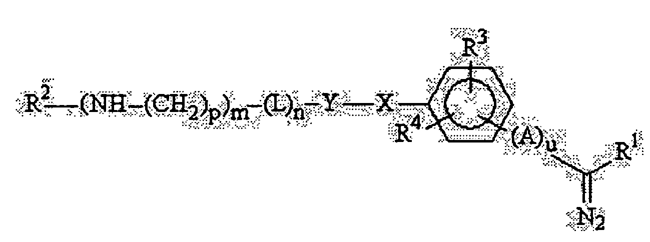 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², - CO-NH-(CH₂)₃-(O-CH₂-CH₂)₄-CH₂-NH-R² avec R = alkyle ou aryle,
• A est un bras de liaison comportant au moins une double liaison covalente permettant la conjugaison de la fonction diazo avec le cycle aromatique et u est un nombre entier compris entre 0 et 2, préférentiellement de 0 ou 1,
• -Y-X- représente -CONH-, -NHCO-, -CH₂O-, -CH₂S-,
• m est un nombre entier compris entre 1 et 10, préférentiellement entre 1 et 3, et
• p est un nombre entier compris entre 1 et 10, préférentiellement entre 1 et 3.

37. Procédé, selon l'une quelconque des revendications 35 ou 36, **caractérisé par le fait que**, dans la formule du réactif, p est inférieur ou égal à m.

38. Procédé, selon l'une quelconque des revendications 35 à 37, dans lequel le réactif est de formule (10) : 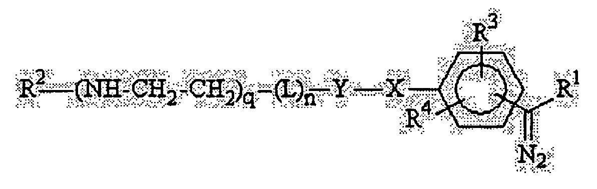 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² -(L)ₙ-Y-X-,OR, SR, NR₂, R, NHCOR, CONHR, COOR, -CO-NH-(CH₂)₃-(O-CH₂-CH₂)₃-CH₂-NH-R², - CO-NH-(CH₂)₃-(O-CH₂-CH₂)₄-CH₂-NH-R² avec R = alkyle ou aryle, et
• q est un nombre entier compris entre 1 et 10, préférentiellement entre 1 et 3

39. Procédé, selon l'une quelconque des revendications 35 à 38, dans lequel le réactif est de formule (11) : 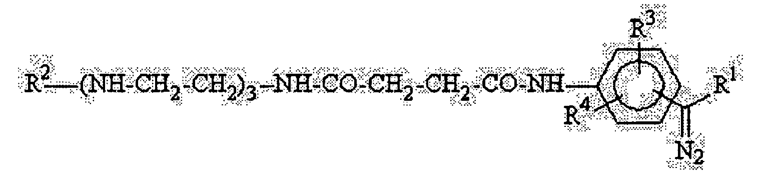 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1, et
• R³ et R⁴ représentent indépendamment l'un de l'autre : H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², - CO-NH-(CH₂)₃-(O-CH₂-CH₂)₄-CH₂-NH-R² avec R = alkyle ou aryle.

40. Procédé, selon l'une quelconque des revendications 35 à 39, **caractérisé par le fait que**, dans le réactif, R² est constitué par un résidu D-Biotine de formule (12):

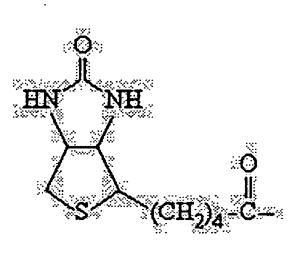

41. Procédé, selon l'une quelconque des revendications 35 à 40, **caractérisé par le fait que**, dans le réactif, R¹ est constitué de : CH₃, et R³ et R⁴ représentent chacun : H.

42. Procédé, selon l'une quelconque des revendications 35 à 41, dans lequel la structure - (L)ₙ- du réactif est constituée par :
• la spermine ou N,N-Bis(3-aminopropyl)-1,4-diaminobutane : NH₂-(CH₂)₃-NH-(CH₂)₄-NH-(CH₂)₃-NH₂, ou
• la spermidine ou N-(3-aminopropyl)-1,4-butandiamine : H₂N-(CH₂)₄-NH-(CH₂)₃-NH₂, ou
• un dérivé contenant un motif alanine : NH₂-CH₂-CH₂-COOH.

43. Procédé de marquage et de purification d'acides nucléiques d'intérêt présents dans un échantillon biologique à traiter, consistant à :
• disposer d'un unique récipient réactionnel,
• introduire dans le récipient réactionnel :
- l'échantillon biologique,
- au moins un réactif de marquage d'acides nucléiques, le réactif de marquage stable à la température est mis en contact en solution homogène, dans un tampon aqueux, et est de formule (13) : 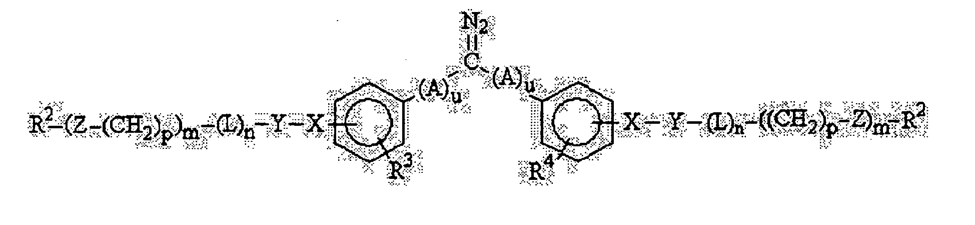 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre : H, NO₂, Cl, Br, F, I, R² - (L)ₙ-Y-X-, OR, SR, NR₂, R, NHCOR, CONHR, COOR, -CO-NH-(CH₂)₃-(O-CH₂-CH₂)₃-CH₂-NH-R², -CO-NH-(CH₂)₃-(O-CH₂-CH₂)₄-CH₂-NH-R² avec R = alkyle ou aryle,
• A est un bras de liaison comportant au moins une double liaison covalente permettant la conjugaison de la fonction diazo avec le cycle aromatique et u est un nombre entier compris entre 0 et 2, préférentiellement de 0 ou 1,
• -Y-X- représente -CONH-, -NHCO-, -CH₂O-, -CH₂S-,
• -Z- représente -NH-, -NHCO-, -CONH- ou -O-,
• m est un nombre entier compris entre 1 et 10, préférentiellement entre 1 et 3, et
• p est un nombre entier compris entre 1 et 10, préférentiellement entre 1 et 3,
- au moins un support solide permettant l'adsorption desdits acides nucléiques,
- tout ingrédient nécessaire au marquage des acides nucléiques et/ou à l'immobilisation desdits acides nucléiques sur le support,
• incuber le contenu du récipient réactionnel, et
• isoler les acides nucléiques ainsi marqués.

44. Procédé, selon la revendication 43, dans lequel le réactif est de formule (16) : 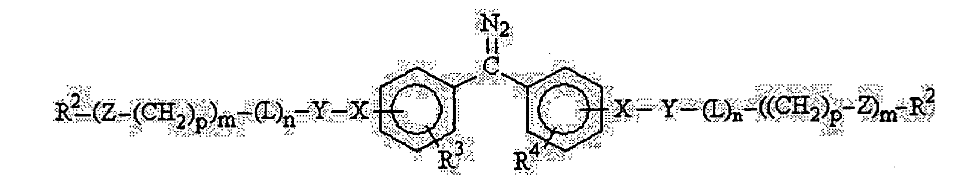 dans laquelle :
• R¹ représente H ou un groupe alkyle, aryle ou aryle substitué,
• R² représente un marqueur détectable ou au moins deux marqueurs détectables reliés entre eux par au moins une structure multimérique,
• L est un bras de liaison comportant un enchaînement linéaire d'au moins deux liaisons covalentes et n un nombre entier égal à 0 ou 1,
• R³ et R⁴ représentent indépendamment l'un de l'autre: H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², - CO-NH- (CH₂)₃-(O-CH₂-CH₂)₄-CH₂-NH-R² avec R = alkyle ou aryle,
• -Y-X- représente -CONH-, -NHCO-, -CH₂O-, -CH₂S-,
• -Z- représente -NH-, -NHCO-, -CONH- ou -O-,
• m est un nombre entier compris entre 1 et 10, préférentiellement entre 1 et 3, et
• p est un nombre entier compris entre 1 et 10, préférentiellement entre 1 et 3.

45. Procédé, selon l'une quelconque des revendications 35 à 44, **caractérisé par le fait que** le constituant L du réactif comprend un motif -(O-CH₂-CH₂)-, répété de 1 à 20 fois, préférentiellement de 1 à 10 fois, et encore plus préférentiellement de 2 à 5 fois, -Z- étant alors représenté par -NH-, -NHCO- ou -CONH-.

46. Procédé, selon l'une quelconque des revendications 1 à 45, **caractérisé par le fait que** le support solide est constitué par des particules de silice.

47. Procédé, selon l'une quelconque des revendications 1 à 46, **caractérisé par le fait que** le support solide est constitué par des particules magnétiques recouvertes de silice.

48. Procédé, selon l'une quelconque des revendications 46 ou 47, **caractérisé par le fait que** les particules de silice constituant le support solide ont des dimensions de particules comprises entre 0,1 et 500 µm et préférentiellement entre 1 et 200 µm.

49. Procédé, selon l'une quelconque des revendications 1 à 48, **caractérisé par le fait qu'**un des ingrédients supplémentaires permettant le marquage est consitué par un alcool de préférence de l'Isopropanol.

50. Procédé, selon la revendication 49, **caractérisé par le fait que** l'Isopropanol constitue 70 % v/v du mélange final.

51. Procédé, selon l'une quelconque des revendications 1 à 50, **caractérisé par le fait qu'**un des ingrédients supplémentaires permettant la libération cellulaire et donc l'adsorption des acides nucléiques sur le support solide est consitué par un agent chaotropique.

52. Procédé, selon la revendication 51, **caractérisé par le fait que** l'agent chaotropique employé est un sel de guanidium, de l'iodure de sodium, de l'iodure de potatium, l'(iso)thiocyanate de sodium, l'urée ou des mélanges de ces dérivés.

53. Procédé, selon la revendication 52, **caractérisé par le fait que** le sel de guanidium employé est l'(iso)thiocyanate de guanidium.

54. Procédé, selon l'une quelconque des revendications 1 à 53, **caractérisé par le fait que** l'on sépare les complexes phase solide-acide nucléique des liquides par sédimention et rejet du surnageant et ensuite lavage des complexes par un tampon de lavage contenant une substance chaotropique.

55. Procédé, selon la revendication 54, **caractérisé par le fait que** les complexes phase solide-acide nucléique, lavés par le tampon de lavage, sont encore lavés ensuite à l'aide d'un ou de plusieurs solvants organiques, et ensuite soumis à un séchage.

56. Procédé, selon la revendication 55, **caractérisé par le fait que** l'acide nucléique présent dans les complexes phase solide-acide nucléique, les complexes étant lavés et séchés, est élué au moyen d'un tampon d'élution.

57. Procédé, selon l'une quelconque des revendications 1 à 53, **caractérisé par le fait que** les complexes phase solide-acide nucléique ainsi obtenus sont mis au contact avec un mélange dans lequel les composants sont présents en vue d'amplifier l'acide nucléique, soit fixé à ladite phase solide, soit élué de celle-ci.

58. Procédé, selon l'une quelconque des revendications 1 à 57, **caractérisé par le fait que** l'étape d'incubation consiste à maintenir l'échantillon traité pendant 5 à 45 minutes, préférentiellement pendant 15 à 35 minutes, et encore plus préférentiellement pendant 25 minutes à une température comprise entre 45 et 85 °C, préférentiellement entre 55 à 75°C, et encore plus préférentiellement à 65°C.

59. Procédé, selon la revendication 58, **caractérisé par le fait qu'**après l'étape d'incubation, l'échantillon est ramené à la température ambiante pendant au moins quelques minutes, préférentiellement 5 minutes.

60. Procédé de marquage et de purification, selon l'une quelconque des revendications 21, 22, 28, 35 ou 43, **caractérisé en ce que** les acides nucléiques traités sont constitués par des ADN et/ou ARN, simple brin et/ou double brins, synthétiques et/ou naturels.

## Claims

1. A process for labeling and purification of nucleic acids of interest which are present in a biological sample to be treated, consisting in:
• taking a single reaction vessel,
• introducing into the reaction vessel:
- the biological sample,
- at least one labeling reagent for nucleic acids, said labeling reagent being stable to heat and of formula (0): 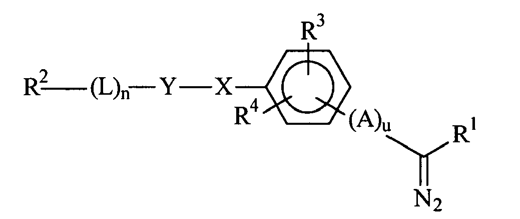 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1,
• R³ and R⁴ independently of one another represent: H, NO₂, Cl, Br, F, I, R² - (L)ₙ-Y-X-, OR, SR, NR₂, R, NHCOR, CONHR or COOR with R = alkyl or aryl,
• A is a linking arm comprising at least one covalent double bond enabling the conjugation of the diazo group with the aromatic ring and u is a whole number lying between 0 and 2, preferably 0 or 1, and
• Y-X- represents -CONH-, -NHCO-, -CH₂O- or -CH₂S-.
- at least one solid support enabling the adsorption of said nucleic acids,
- any ingredient necessary for the labeling of the nucleic acids and/or for the immobilization of said nucleic acids on the support,
• incubating the contents of the reaction vessel, and
• isolating the nucleic acids thus labeled.

2. The labeling and purification process as claimed in claim 1, **characterized in that** the nucleic acids treated consist of single-strand and/or double-strand, synthetic and/or natural DNA and/or RNA.

3. The labeling and purification process as claimed in any one of claims 1 or 2, **characterized in that** the introduction of the labeling reagent enables:
- the fragmentation of the nucleic acids in a non-specific manner to generate a plurality of nucleic acid fragments, and
- the labeling of a plurality of these fragments at the terminal phosphate situated at the 3' and/or 5' end, said terminal phosphate having been liberated during the fragmentation.

4. The labeling and purification process as claimed in claim 3, **characterized in that** the labeling of the 3' or 5' end of a nucleic acid fragment is performed by attachment of a reactive group borne by a label onto the phosphate in the 2' position, 3' position or cyclic 2'-3'-mono-phosphate position, with reference to the ribose.

5. The labeling and purification process as claimed in claim 3, **characterized in that** the fragmentation and/or labeling of the 3' or 5' end of a nucleic acid fragment is performed by attachment of a nucleophilic, electrophilic or halide group borne by a label onto the phosphate in the 2' position, 3' position or cyclic 2'-3'-monophosphate position, with reference to the ribose.

6. The process as claimed in any one of claims 3 to 5, **characterized in that** the fragmentation of the nucleic acids is performed by:
- enzymatic means (nucleases),
- chemical means (metal cations, such as Mg⁺⁺, Mn⁺⁺, Cu⁺⁺, Co⁺⁺ and/or Zn⁺⁺ ions, whether or not in combination with a chemical catalyst, for example N-methylimidazole, or any chemical molecule having an affinity for RNA and bearing an imidazole nucleus or a substituted analog) or
- physical means (by sonication or by radiation).

7. The process as claimed in any one of claims 3 to 6, **characterized in that** the labeling of the 3' or 5' end of a fragment of RNA is performed by attachment of a molecule R-X, where R consists of the label and X is the binding agent between the label and the RNA, such as a hydroxyl, amine, hydrazine, alkoxylamine, alkyl halide, phenylmethyl halide, iodoacetamide or maleimide group onto the phosphate linked to the 2' position, 3' position or the cyclic 2'-3'-monophosphate position of the ribose.

8. The process as claimed in any one of claims 3 to 7, **characterized in that** the labeling on the phosphate group is effected by means of 5-(bromomethyl)-fluorescein.

9. The labeling and purification process as claimed in any one of claims 1 or 2, **characterized in that** the labeling reagent is contacted with the nucleic acids in homogenous solution, in an essentially aqueous buffer, said labeling reagent being stable to heat and of formula (0).

10. The process as claimed in claim 9, wherein the labeling reagent used is of formula (1): 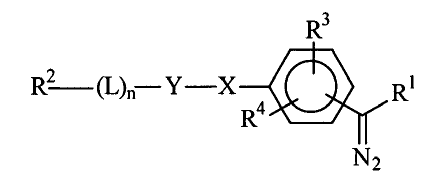 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1,
• R³ and R⁴ independently of one another represent: H, NO₂, Cl, Br, F, I, R² -(L)ₙ-Y-X-, OR, SR, NR₂, R, NHCOR, CONHR or COOR with R = alkyl or aryl, and
• -Y-X- represents -CONH-, -NHCO-, -CH₂O- or -CH₂S-.

11. The process as claimed in any one of claims 9 or 10, wherein the reagent is of formula (2): 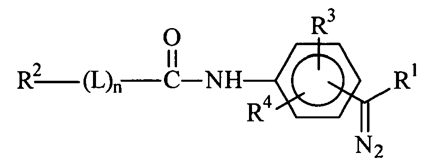 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1, and
• R³ and R⁴ independently of one another represent: H, NO₂, Cl, Br, F, I, R² -(L)ₙ-Y-X-, OR, SR, NR₂, R, NHCOR, CONHR or COOR with R = alkyl or aryl.

12. The process as claimed in any one of claims 9 or 10, wherein the reagent is of formula (3): 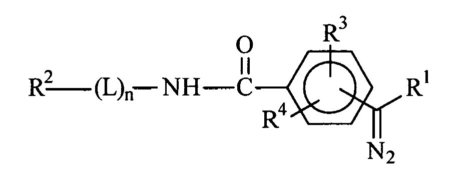 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1, and
• R³ and R⁴ independently of one another represent: H, NO₂, Cl, Br, F, I, R² -(L)ₙ-Y-X-, OR, SR, NR₂, R, NHCOR, CONHR or COOR with R = alkyl or aryl.

13. The process as claimed in any one of claims 9 or 10, wherein the reagent is of formula (4): 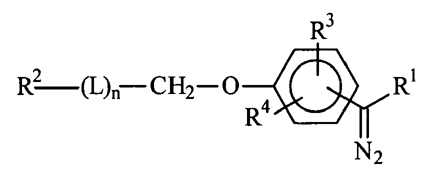 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1, and
• R³ and R⁴ independently of one another represent: H, NO₂, Cl, Br, F, I, R² -(L)ₙ-Y-X-, OR, SR, NR₂, R, NHCOR, CONHR or COOR with R = alkyl or aryl.

14. The process as claimed in claim 9, wherein the reagent is of formula (21): 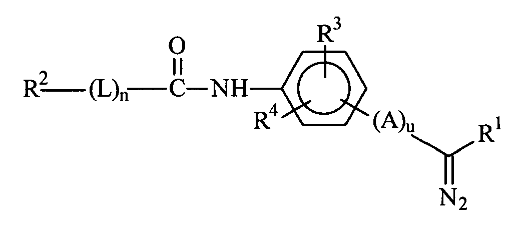 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1,
• A is a linking arm comprising at least one covalent double bond enabling the conjugation of the diazo group with the aromatic ring and u equals 1, and
• R³ and R⁴ independently of one another represent: H, NO₂, Cl, Br, F, I, R² -(L)ₙ-Y-X-, OR, SR, NR₂, R, NHCOR, CONHR or COOR with R = alkyl or aryl.

15. The process as claimed in claim 9, wherein the reagent is of formula (22): 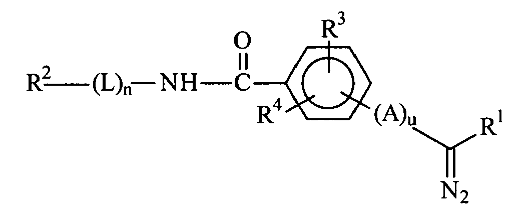 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1,
• A is a linking arm comprising at least one covalent double bond enabling the conjugation of the diazo group with the aromatic ring and u equals 1, and
• R³ and R⁴ independently of one another represent: H, NO₂, Cl, Br, F, I, R² -(L)ₙ-Y-X-, OR, SR, NR₂, R, NHCOR, CONHR or COOR with R = alkyl or aryl.

16. The process as claimed in claim 9, wherein the reagent is of formula (23): 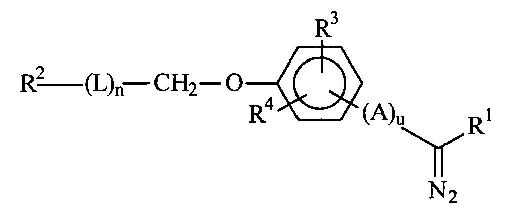 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1,
• A is a linking arm comprising at least one covalent double bond enabling the conjugation of the diazo group with the aromatic ring and u equals 1, and
• R³ and R⁴ independently of one another represent: H, NO₂, Cl, Br, F, I, R² -(L)ₙ Y-X-, OR, SR, NR₂, R, NHCOR, CONHR or COOR with R = alkyl or aryl.

17. The process as claimed in any one of claims 9 to 16, **characterized in that**, wherein the labeling reagent used contains R³ and R⁴ which independently of one another represent: H, NO₂, OCH₃, -CO-NH-(CH₂)₃-(O-CH₂-CH₂)₃-CH₂-NH-R² or -CO-NH-(CH₂)₃-(O-CH₂-CH₂)₄-CH₂-NH-R².

18. The process as claimed in any one of claims 9 to 11, wherein the reagent is of formula (7): 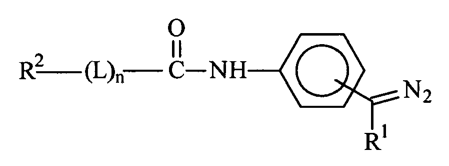 wherein:
• R¹ represents H or an alkyl, aryl or substituted aryl group,
• R² represents a detectable label or at least two detectable labels linked together by at least one multimeric structure, and
• L is a linking arm comprising a linear chain of at least two covalent bonds and n a whole number equal to 0 or 1.

19. The process as claimed in any one of claims 9 or 14, wherein the reagent is of formula (24): 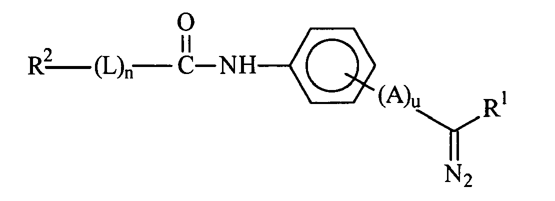 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1, and
• A is a linking arm comprising at least one covalent double bond enabling the conjugation of the diazo group with the aromatic ring and u equals 1.

20. The process as claimed in any one of claims 9 to 19, wherein the structure R²-(L)ₙ- in the reagent is of formula (5): 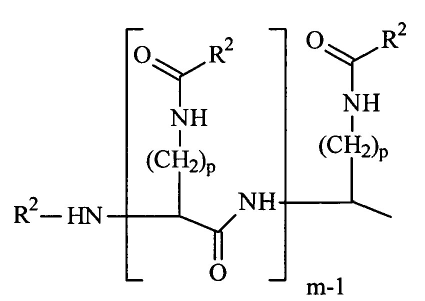 wherein:
• R² represents a detectable label,
• m is a whole number lying between 1 and 100, and
• p is a whole number lying between 1 and 10.

21. A process for labeling and purification of nucleic acids of interest present in a biological sample to be treated, comprising:
• taking a single reaction vessel,
• introducing into the reaction vessel:
- the biological sample,
- at least one labeling reagent for nucleic acids, the labeling reagent being contacted with the nucleic acids in homogenous solution, in an essentially aqueous buffer, said labeling reagent being stable to heat, and of formula (6): 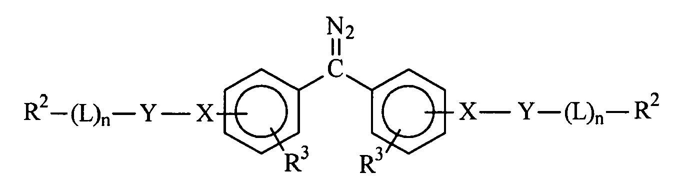 wherein:
• R² represents a detectable label or at least two detectable labels linked together by at least one multimeric structure,
• R³ represents H, NO₂, Cl, Br, F, I, R² -(L)ₙ-Y-X-, OR, SR, NR₂, R, NHCOR, CONHR or COOR with R = alkyl or aryl,
• L is a linking arm comprising a linear chain of at least two covalent bonds and n a whole number equal to 0 or 1, and
• -Y-X- represents -CONH-, -NHCO-, -CH₂O- or -CH₂S-,
- at least one solid support enabling the adsorption of said nucleic acids,
- any ingredient necessary for the labeling of the nucleic acids and/or for the immobilization of said nucleic acids on the support,
• incubating the contents of the reaction vessel, and
• isolating the nucleic acids thus labeled.

22. A process for labeling and purification of nucleic acids of interest present in a biological sample to be treated, comprising:
• taking a single reaction vessel,
• introducing into the reaction vessel:
- the biological sample,
- at least one labeling reagent for nucleic acids, the labeling reagent being contacted with the nucleic acids in homogenous solution, in an essentially aqueous buffer, said labeling reagent being stable to heat, and of formula (25): 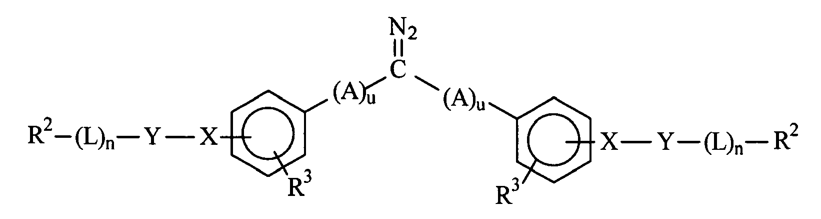 wherein:
• R² represents a detectable label or at least two detectable labels linked together by at least one multimeric structure,
• R³ represents H, NO₂, Cl, Br, F, I, R² -(L)ₙ-Y-X-, OR, SR, NR₂, R, NHCOR, CONHR or COOR with R = alkyl or aryl,
• L is a linking arm comprising a linear chain of at least two covalent bonds and n a whole number equal to 0 or 1,
• A is a linking arm comprising at least one covalent double bond enabling the conjugation of the diazo group with the aromatic ring and u equals 1, and
• -Y-X- represents -CONH-, -NHCO-, -CH₂O- or -CH₂S-,
- at least one solid support enabling the adsorption of said nucleic acids,
- any ingredient necessary for the labeling of the nucleic acids and/or for the immobilization of said nucleic acids on the support,
• incubating the contents of the reaction vessel, and
• isolating the nucleic acids thus labeled.

23. The process as claimed in any one of claims 9 to 11, wherein the reagent is of formula (14): 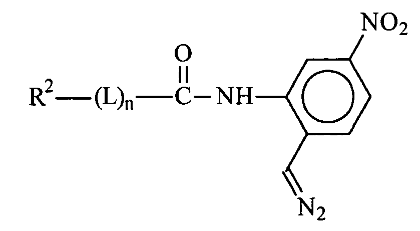 wherein:
• R² represents a detectable label or at least two detectable labels linked together by at least one multimeric structure, and
• L is a linking arm comprising a linear chain of at least two covalent bonds and n a whole number equal to 0 or 1.

24. The process as claimed in any one of claims 9 or 14, wherein the reagent is of formula (26): 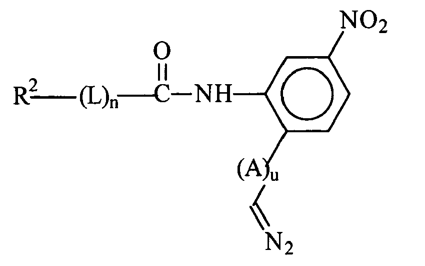 wherein:
• R² represents a detectable label or at least two detectable labels linked together by at least one multimeric structure,
• A is a linking arm comprising at least one covalent double bond enabling the conjugation of the diazo group with the aromatic ring and u equals 1, and
• L is a linking arm comprising a linear chain of at least two covalent bonds and n a whole number equal to 0 or 1.

25. The process as claimed in any one of claims 9 to 11, wherein the reagent is of formula (15): 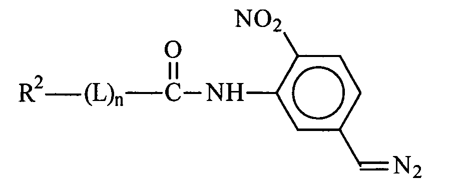 wherein:
• R² represents a detectable label or at least two detectable labels linked together by at least one multimeric structure, and
• L is a linking arm comprising a linear chain of at least two covalent bonds and n a whole number equal to 0 or 1.

26. The process as claimed in any one of claims 9 or 14, wherein the reagent is of formula (27): 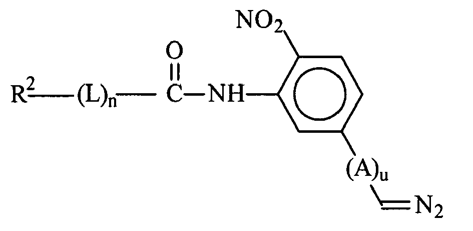 wherein:
• R² represents a detectable label or at least two detectable labels linked together by at least one multimeric structure,
• A is a linking arm comprising at least one covalent double bond enabling the conjugation of the diazo group with the aromatic ring and u equals 1, and
• L is a linking arm comprising a linear chain of at least two covalent bonds and n a whole number equal to 0 or 1.

27. The process as claimed in any one of claims 9 to 26, **characterized in that** the constituent L of the reagent contains a -(O-CH₂-CH₂)- moiety, repeated from 1 to 20 times, preferably from 1 to 10 times, and still more preferably from 2 to 5 times.

28. A process for labeling and purification of nucleic acids of interest present in a biological sample to be treated, comprising:
• taking a single reaction vessel,
• introducing into the reaction vessel:
- the biological sample,
- at least one labeling reagent for nucleic acids, the labeling reagent enabling the labeling and the fragmentation a single or double-strand nucleic acid according to the following steps:
o fragmenting the nucleic acid,
o attaching a label onto at least one of the fragments by means of a labeling reagent selected from the compounds of formula (19): 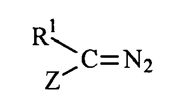 wherein:
• R¹ represents H or an alkyl, aryl or substituted aryl group, and
• Z contains a detectable label,
said reagent coupling covalently and mainly onto at least one phosphate of said fragment,
- at least one solid support enabling the adsorption of said nucleic acids,
- any ingredient necessary for the labeling of the nucleic acids and/or for the immobilization of said nucleic acids on the support,
• incubating the contents of the reaction vessel, and
• isolating the nucleic acids thus labeled.

29. The process as claimed in claim 28, **characterized in that** the labeling reagent is selected from the compounds of formula (20): 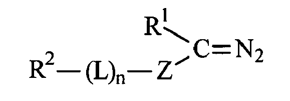 wherein:
• R¹ represents H or an alkyl, aryl or substituted aryl group,
• R² is a detectable label or at least two detectable labels linked together by at least one multimeric structure,
• L is a linking arm comprising a linear chain of at least two covalent bonds and n is equal to 0 or 1, and
• Z is selected from: 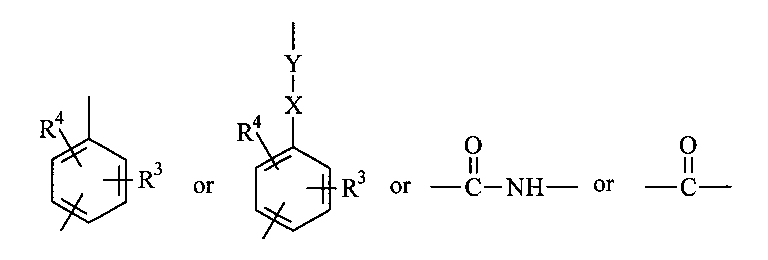
wherein:
■ R³ and R⁴ independently of one another represent: H, NO₂, Cl, Br, F, I, R² -(L)ₙ-Y-X-, OR, SR, NR₂, R, NHCOR, CONHR or COOR with R = alkyl or aryl, and
■ -Y-X- represents -CONH-, -NHCO-, -CH₂O- or -CH₂S-.

30. The process as claimed in claim 28, **characterized in that** Z is:

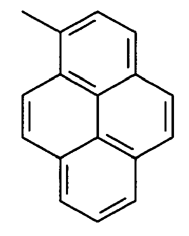

31. The process as claimed in any one of claims 28 to 30, **characterized in that** the fragmentation and the labeling are performed in two steps.

32. The process as claimed in any one of claims 28 to 30, **characterized in that** the fragmentation and the labeling are performed in one step.

33. The process as claimed in any one of claims 28 to 32, **characterized in that** the labeling is performed in essentially aqueous homogeneous solution.

34. The process as claimed in any one of claims 28 to 33, **characterized in that** the fragmentation is performed by enzymatic, physical or chemical means.

35. A process for labeling and purification of nucleic acids of interest present in a biological sample to be treated, comprising:
• taking a single reaction vessel,
• introducing into the reaction vessel:
- the biological sample,
- at least one labeling reagent for nucleic acids, the labeling reagent being contacted in homogeneous solution, in an essentially aqueous buffer, said labeling reagent being stable to heat and of formula (8): 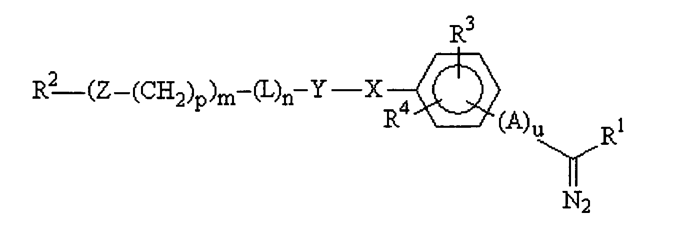 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1,
• R³ and R⁴ independently of one another represent: H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R² or -CO-NH-(CH₂)₃-(O-CH₂-CH₂)₄-CH₂-NH-R² with R = alkyl or aryl,
• A is a linking arm comprising at least one covalent double bond enabling the conjugation of the diazo group with the aromatic ring and u is a whole number lying between 0 and 2, preferably 0 or 1,
• -Y-X- represents -CONH-, -NHCO-, -CH₂O- or -CH₂S-,
• -Z- represents -NH-, -NHCO-, -CONH- or -O-,
• m is a whole number lying between 1 and 10, preferably between 1 and 3, and
• p is a whole number lying between 1 and 10, preferably between 1 and 3,
- at least one solid support enabling the adsorption of said nucleic acids,
- any ingredient necessary for the labeling of the nucleic acids and/or for the immobilization of said nucleic acids on the support,
• incubating the contents of the reaction vessel, and
• isolating the nucleic acids thus labeled.

36. The process as claimed in claim 35, wherein the reagent is of formula (9): 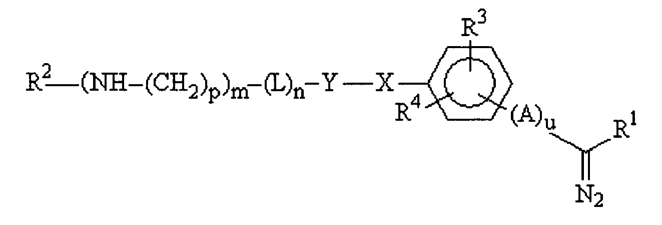 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1,
• R³ and R⁴ independently of one another represent: H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R² or -CO-NH-(CH₂)₃-(O-CH₂-CH₂)₄-CH₂-NH-R² with R = alkyl or aryl,
• A is a linking arm comprising at least one covalent double bond enabling the conjugation of the diazo group with the aromatic ring and u is a whole number lying between 0 and 2, preferably 0 or 1,
• -Y-X- represents -CONH-, -NHCO-, -CH₂O- or -CH₂S-,
• m is a whole number lying between 1 and 10, preferably between 1 and 3, and
• p is a whole number lying between 1 and 10, preferably between 1 and 3.

37. The process as claimed in any one of claims 35 or 36, **characterized in that**, in the formula of the reagent, p is less than or equal to m.

38. The process as claimed in any one of claims 35 to 37, wherein the reagent is of formula (10): 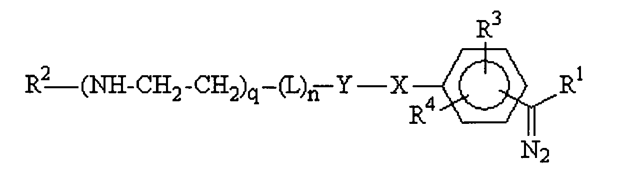 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1,
• R³ and R⁴ independently of one another represent: H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R² or -CO-NH-(CH₂)₃-(O-CH₂-CH₂)₄-CH₂-NH-R² with R = alkyl or aryl, and
• q is a whole number lying between 1 and 10, preferably between 1 and 3

39. The process as claimed in any one of claims 35 to 38, wherein the reagent is of formula (11): 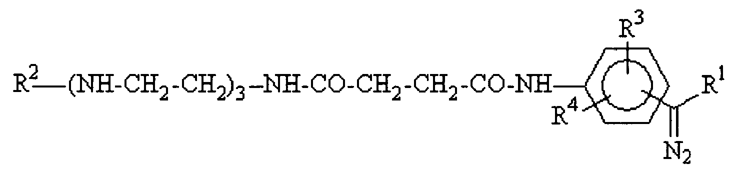 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1, and
• R³ and R⁴ independently of one another represent: H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², or -CO-NH-(CH₂)₃-(O-CH₂-CH₂)₄-CH₂-NH-R² with R = alkyl or aryl.

40. The process as claimed in any one of claims 35 to 39, **characterized in that**, in the reagent, R² comprises a D-Biotin residue of formula (12):

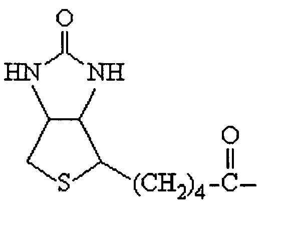

41. The process as claimed in any one of claims 35 to 40, **characterized in that**, in the reagent, R¹ comprises: CH₃, and R³ and R⁴ each represent: H.

42. The process as claimed in any one of claims 35 to 41, wherein the structure -(L)ₙ-of the reagent comprises:
• spermine or N,N'-bis(3-aminopropyl)-1,4-diaminobutane: NH₂-(CH₂)₃-NH-(CH₂)₄-NH-(CH₂)₃-NH₂, or
• spermidine or N-(3-aminopropyl)-1,4-butanediamine: H₂N-(CH₂)₄-NH-(CH₂)₃-NH₂, or
• a derivative containing an alanine moiety: NH₂-CH₂-CH₂-COOH.

43. A process for labeling and purification of nucleic acids of interest present in a biological sample to be treated, comprising:
• taking a single reaction vessel,
• introducing into the reaction vessel:
- the biological sample,
- at least one labeling reagent for nucleic acids, the heat-stable labeling reagent is contacted in homogenous solution, in an aqueous buffer, and is of formula (13): 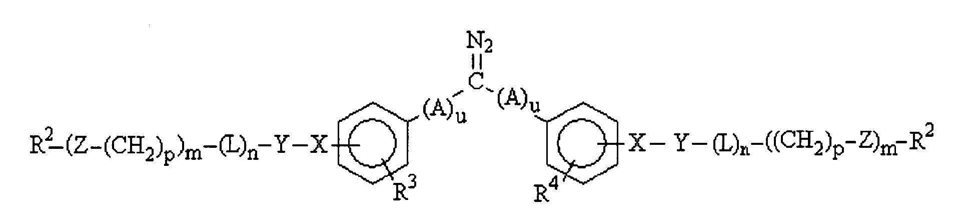 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1,
• R³ and R⁴ independently of one another represent: H, NO₂, Cl, Br, F, I, R² - (L)ₙ-Y-X-, OR, SR, NR₂, R, NHCOR, CONHR, COOR, -CO-NH-(CH₂)₃-(O-CH₂-CH₂)₃-CH₂-NH-R² or -CO-NH-(CH₂)₃-(O-CH₂-CH₂)₄-CH₂-NH-R² with R = alkyl or aryl,
• A is a linking arm comprising at least one covalent double bond enabling the conjugation of the diazo group with the aromatic ring and u is a whole number lying between 0 and 2, preferably 0 or 1,
• -Y-X- represents -CONH-, -NHCO-, -CH₂O- or -CH₂S-,
• -Z- represents -NH-, -NHCO-, -CONH- or -O-,
• m is a whole number lying between 1 and 10, preferably between 1 and 3, and
• p is a whole number lying between 1 and 10, preferably between 1 and 3,
- at least one solid support enabling the adsorption of said nucleic acids,
- any ingredient necessary for the labeling of the nucleic acids and/or for the immobilization of said nucleic acids on the support,
• incubating the contents of the reaction vessel, and
• isolating the nucleic acids thus labeled.

44. The process as claimed in claim 43, wherein the reagent is of formula (16): 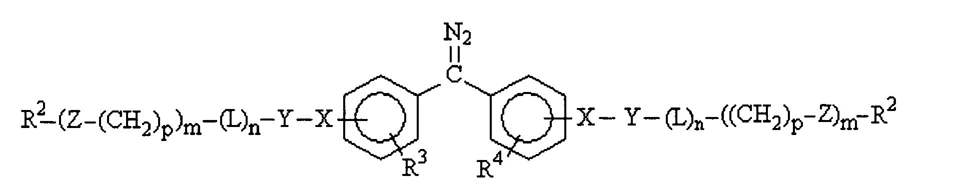 wherein:
• R¹ represents H or an alkyl, aryl or substituted aryl group,
• R² represents a detectable label or at least two detectable labels linked together by at least one multimeric structure,
• L is a linking arm comprising a linear chain of at least two covalent bonds and n a whole number equal to 0 or 1,
• R³ and R⁴ independently of one another represent: H, NO₂, Cl, Br, F, I, R² -(L)ₙ-Y-X-, OR, SR, NR₂, R, NHCOR, CONHR, COOR, -CO-NH-(CH₂)₃-(O-CH₂-CH₂)₃-CH₂-NH-R², or -CO-NH-(CH₂)₃-(O-CH₂-CH₂)₄-CH₂-NH-R² with R = alkyl or aryl,
• -Y-X- represents -CONH-, -NHCO-, -CH₂O- or -CH₂S-,
• -Z- represents -NH-, -NHCO-, -CONH- or -O-,
• m is a whole number lying between 1 and 10, preferably between 1 and 3, and
• p is a whole number lying between 1 and 10, preferably between 1 and 3.

45. The process as claimed in any one of claims 35 to 44, **characterized in that** the constituent L of the reagent contains an -(O-CH₂-CH₂)- moiety, repeated from 1 to 20 times, preferably from 1 to 10 times, and still more preferably from 2 to 5 times, -Z- then being represented by -NH-, -NHCO- or -CONH-.

46. The process as claimed in any one of claims 1 to 45, **characterized in that** the solid support consists of particles of silica.

47. The process as claimed in any one of claims 1 to 46, **characterized in that** the solid support consists of magnetic particles covered with silica.

48. The process as claimed in any one of claims 46 or 47, **characterized in that** the particles of silica comprising the solid support have particle sizes lying between 0.1 and 500 µm and preferably between 1 and 200 µm.

49. The process as claimed in any one of claims 1 to 48, **characterized in that** one of the supplementary ingredients enabling the labeling consists of an alcohol preferably Isopropanol.

50. The process as claimed in claim 49, **characterized in that** Isopropanol comprises 70% v/v of the final mixture.

51. The process as claimed in any one of claims 1 to 50, **characterized in that** one of the supplementary ingredients enabling the cell liberation and thus the adsorption of the nucleic acids onto the solid support consists of a chaotropic agent.

52. The process as claimed in claim 51, **characterized in that** the chaotropic agent used is a guanidium salt, sodium iodide, potassium iodide, sodium (iso)thiocyanate, urea or mixtures of these derivatives.

53. The process as claimed in claim 52, **characterized in that** the guanidium salt used is guanidium (iso)thiocyanate.

54. The process as claimed in any one of claims 1 to 53, **characterized in that** the solid phase-nucleic acid complexes are separated from the liquids by sedimentation and rejection of the supernatant, then washing of the complexes with a washing buffer containing a chaotropic substance.

55. The process as claimed in claim 54, **characterized in that** the solid phase-nucleic acid complexes, after washing with the washing buffer, are then washed again with one or several organic solvents, and then subjected to a drying process.

56. The process as claimed in claim 55, **characterized in that** the nucleic acid present in the solid phase-nucleic acid complexes, after washing and drying of the complexes, is eluted by means of an elution buffer.

57. The process as claimed in any one of claims 1 to 53, **characterized in that** the solid phase-nucleic acid complexes thus obtained are contacted with a mixture wherein the components are present for the purpose of amplifying the nucleic acid, whether attached to said solid phase, or eluted therefrom.

58. The process as claimed in any one of claims 1 to 57, **characterized in that** the incubation step comprises maintaining the treated sample for 5 to 45 minutes, preferably for 15 to 35 minutes, and still more preferably for 25 minutes at a temperature lying between 45 and 85 °C, preferably between 55 and 75°C, and still more preferably at 65°C.

59. The process as claimed in claim 58, **characterized in that** after the incubation step, the sample is brought back to ambient temperature for a least a few minutes, preferably 5 minutes.

60. The labeling and purification process as claimed in any one of claims 21, 22, 28, 35 or 43, **characterized in that** the nucleic acids treated consist of single-strand and/or double-strand, synthetic and/or natural DNA and/or RNA.

## Patentansprüche

1. Verfahren zur Markierung und Reinigung von Nukleinsäuren von Interesse, die in einer zu behandelnden biologischen Probe vorliegen, das folgendes umfaßt:
• Verfügen über ein einziges Reaktionsgefäß,
• Einbringen in dieses Reaktionsgefäß von folgendem:
- die biologische Probe,
- mindestens einen Reaktanten für die Markierung von Nukleinsäuren, wobei dieser Markierungsreaktant temperaturstabil ist und die Formel (0) aufweist: 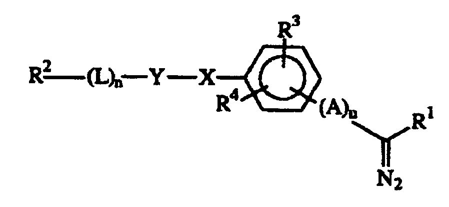 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet,
• R³ und R⁴ unabhängig voneinander H, NO₂, Cl, Br, F, I, R² - (L)ₙ-Y-X-, OR, SR, NR₂, R, NHCOR, CONHR, COOR, wobei R = Alkyl oder Aryl, bedeuten,
• A ein Bindeglied bedeutet, das mindestens eine kovalente Doppelbindung enthält, die die Konjugation der Diazofunktion mit dem aromatischen Ring gestattet, und u eine ganze Zahl zwischen 0 und 2 bedeutet und vorzugsweise 0 oder 1 ist, und
• Y-X- -CONH-, -NHCO-, -CH₂O-, -CH₂S- bedeutet,
- mindestens einen festen Träger, der die Adsorption der genannten Nukleinsäuren ermöglicht,
- beliebige Bestandteile, die für die Markierung der Nukleinsäuren und/oder die Immobilisierung der genannten Nukleinsäuren auf dem Träger erforderlich sind,
• Inkubieren des Inhalts des Reaktionsgefäßes, und
• Isolieren der so markierten Nukleinsäuren.

2. Markierungs- und Aufreinigungsverfahren nach Anspruch 1, **dadurch gekennzeichnet, daß** die behandelten Nukleinsäuren aus den einzelsträngigen und/oder doppelsträngigen, synthetischen und/oder natürlichen DNAs und/oder RNAs bestehen.

3. Markierungs- und Aufreinigungsverfahren nach einem der Ansprüche 1 oder 2, **dadurch gekennzeichnet, daß** das Einbringen des Markierungsreaktanten folgendes ermöglicht:
- eine unspezifische Fragmentierung der Nukleinsäuren, um eine Mehrzahl von Nukleinsäurefragmenten zu erhalten, und
- die Markierung einer Mehrzahl dieser Fragmente am endständigen Phosphat, das sich am 3'-und/oder 5'-terminalen Ende befindet, wobei dieses endständige Phosphat bei der Fragmentierung freigesetzt worden ist.

4. Markierungs- und Aufreinigungsverfahren nach Anspruch 3, **dadurch gekennzeichnet, daß** die Markierung des 3'- oder des 5'-terminalen Endes eines Nukleinsäurefragments **dadurch** erfolgt, daß eine reaktionsfähige Funktion, die von einem Marker getragen wird, an dem Phosphat in 2'-Stellung, in 3'-Stellung oder in 2'-3'-Stellung des cyclischen Monophosphats in Bezug auf die Ribose angebracht wird.

5. Markierungs- und Aufreinigungsverfahren nach Anspruch 3, **dadurch gekennzeichnet, daß** die Fragmentierung und/oder Markierung des 3'- oder des 5'-terminalen Endes eines Nukleinsäurefragments **dadurch** erfolgt, daß eine nukleophile, elektrophile Halogenidfunktion, die von einem Marker getragen wird, an dem Phosphat in 2'-Stellung, in 3'-Stellung oder in 2'-3'-Stellung des cyclischen Monophosphats in Bezug auf die Ribose angebracht wird.

6. Verfahren nach einem der Ansprüche 3 bis 5, **dadurch gekennzeichnet, daß** die Fragmentierung der Nukleinsäuren folgermaßen erfolgt:
- auf enzymatischem Weg (Nukleasen),
- auf chemischem Weg (Metallkationen wie Mg⁺⁺-, Mn⁺⁺-, Cu⁺⁺-, Co⁺⁺- und/oder Zn⁺⁺-Ionen, die gegebenenfalls mit einem chemischen Katalysator verbunden sind, zum Beispiel N-Methylimidazol, oder beliebige chemische Moleküle mit einer Affinität für RNA, die einen Imidazolkern oder ein substituiertes Analog tragen), oder
- auf physikalischem Weg (durch Ultraschallbehandlung oder Strahlung).

7. Verfahren nach einem der Ansprüche 3 bis 6, **dadurch gekennzeichnet, daß** die Markierung des 3'-oder des 5'-terminalen Endes eines RNA-Fragments **dadurch** erfolgt, daß man ein Molekül R-X, wobei R aus dem Marker besteht und X das Bindemittel zwischen dem Marker und der RNA bedeutet, wie eine Hydroxyl-, Amin-, Hydrazin-, Alkoxylamin-, Alkylhalogenid-, Phenylmethylhalogenid-, Iodacetamid-, Maleimidgruppe, an dem Phosphat in 2'-Stellung, in 3'-Stellung oder in 2'-3'-Stellung des cyclischen Monophosphats in Bezug auf die Ribose anbringt.

8. Verfahren nach einem der Ansprüche 3 bis 7, **dadurch gekennzeichnet, daß** die Markierung von der Phosphatgruppe mittels 5-(Brommethyl)fluorescein erfolgt.

9. Markierungs- und Aufreinigungsverfahren nach einem der Ansprüche 1 oder 2, **dadurch gekennzeichnet, daß** der Markierungsreaktant mit den Nukleinsäuren in homogener Lösung in einem im wesentlichen wäßrigen Puffer erfolgt, wobei der Markierungsreaktant temperaturstabil ist und die Formel (0) aufweist.

10. Verfahren nach Anspruch 9, bei dem der verwendete Markierungsreaktant die Formel (I) aufweist: 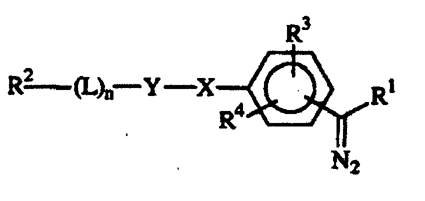 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet,
• R³ und R⁴ unabhängig voneinander H, NO₂, Cl, Br, F, I, R² - (L)ₙ-Y-X-, OR, SR, NR₂, R, NHCOR, CONHR, COOR, wobei R = Alkyl oder Aryl, bedeuten,
• Y-X -CONH-, -NHCO-, -CH₂O-, -CH₂S- bedeutet.

11. Verfahren nach einem der Ansprüche 9 oder 10, bei dem der Reaktant die Formel (2) aufweist: 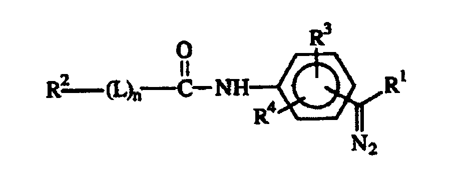 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet, und
• R³ und R⁴ unabhängig voneinander H, NO₂, Cl, Br, F, I, R² - (L)ₙ-Y-X-, OR, SR, NR₂, R, NHCOR, CONHR, COOR, wobei R = Alkyl oder Aryl, bedeuten.

12. Verfahren nach einem der Ansprüche 9 oder 10, bei dem der Reaktant die Formel (3) aufweist: 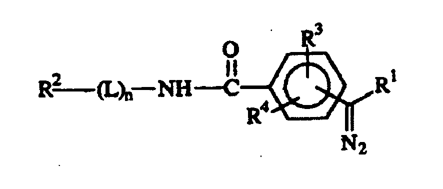 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet, und
• R³ und R⁴ unabhängig voneinander H, NO₂, Cl, Br, F, I, R² - (L)ₙ-Y-X-, OR, SR, NR₂, R, NHCOR, CONHR, COOR, wobei R = Alkyl oder Aryl, bedeuten.

13. Verfahren nach einem der Ansprüche 9 oder 10, bei dem der Reaktant die Formel (4) aufweist: 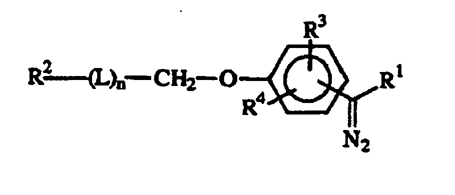 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet, und
• R³ und R⁴ unabhängig voneinander H, NO₂, Cl, Br, F, I, R² - (L)ₙ-Y-X-, OR, SR, NR₂, R, NHCOR, CONHR, COOR, wobei R = Alkyl oder Aryl, bedeuten.

14. Verfahren nach Anspruch 9, bei dem der Reaktant die Formel (21) aufweist: 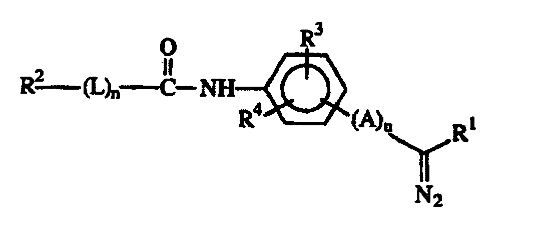 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet,
• A ein Bindeglied bedeutet, das mindestens eine kovalente Doppelbindung enthält, die die Konjugation der Diazofunktion mit dem aromatischen Ring gestattet, und u gleich 1 ist
• R³ und R⁴ unabhängig voneinander H, NO₂, Cl, Br, F, I, R² - (L)ₙ-Y-X-, OR, SR, NR₂, R, NHCOR, CONHR, COOR, wobei R = Alkyl oder Aryl, bedeuten.

15. Verfahren nach Anspruch 9, bei dem der Reaktant die Formel (22) aufweist: 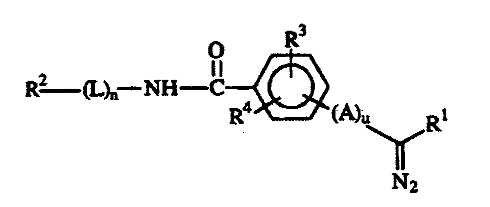 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet,
• A ein Bindeglied bedeutet, das mindestens eine kovalente Doppelbindung enthält, die die Konjugation der Diazofunktion mit dem aromatischen Ring gestattet, und u gleich 1 ist, und
• R³ und R⁴ unabhängig voneinander H, NO₂, Cl, Br, F, I, R² - (L)ₙ-Y-X-, OR, SR, NR₂, R, NHCOR, CONHR, COOR, wobei R = Alkyl oder Aryl, bedeuten.

16. Verfahren nach Anspruch 9, bei dem der Reaktant die Formel (23) aufweist: 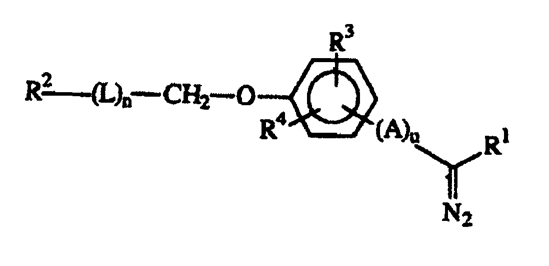 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet,
• A ein Bindeglied bedeutet, das mindestens eine kovalente Doppelbindung enthält, die die Konjugation der Diazofunktion mit dem aromatischen Ring gestattet, und u gleich 1 ist, und
• R³ und R⁴ unabhängig voneinander H, NO₂, Cl, Br, F, I, R² - (L)ₙ-Y-X-, OR, SR, NR₂, R, NHCOR, CONHR, COOR, wobei R = Alkyl oder Aryl, bedeuten.

17. Verfahren nach einem der Ansprüche 9 bis 16, **dadurch gekennzeichnet, daß** darin der verwendete Markierungsreaktant R³ und R⁴ umfaßt, die unabhängig voneinander folgendes bedeuten: H, NO₂, OCH₃, -CO-NH-(CH₂)₃-(O-CH₂-CH₂)₃-CH₂-NH-R², -CO-NH-(CH₂)₃-(O-CH₂-CH₂)₄-CH₂-NH-R².

18. Verfahren nach einem der Ansprüche 9 bis 11, bei dem der Reaktant die Formel (7) aufweist: 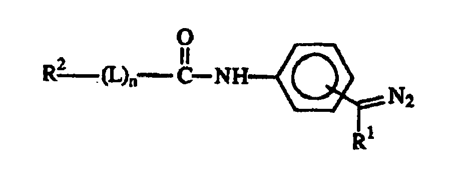 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet, und
• L ein Bindeglied bedeutet, das eine lineare Verknüpfung von mindestens zwei kovalenten Bindungen enthält, und n eine ganze Zahl gleich 0 oder 1 bedeutet.

19. Verfahren nach einem der Ansprüche 9 oder 14, bei dem der Reaktant die Formel (24) aufweist: 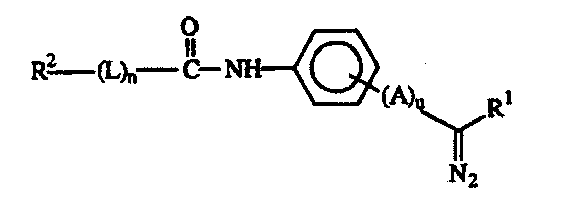 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet, und
• A ein Bindeglied bedeutet, das mindestens eine kovalente Doppelbindung enthält, die die Konjugation der Diazofunktion mit dem aromatischen Ring gestattet, und u gleich 1 ist.

20. Verfahren nach einem der Ansprüche 9 bis 19, bei dem die Struktur R²-(L)ₙ- des Reaktanten die Formel (5) aufweist: 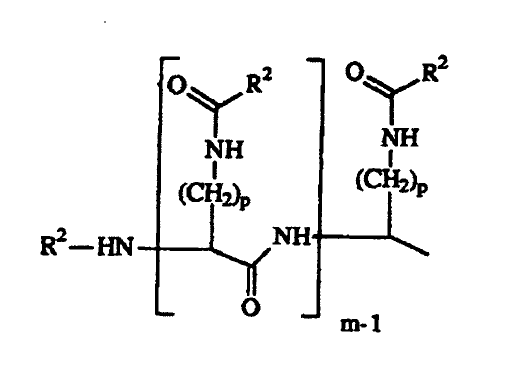 in der
• R² einen nachweisbaren Marker bedeutet,
• m eine ganze Zahl zwischen 1 und 100 bedeutet, und
• p eine ganze Zahl zwischen 1 und 10 bedeutet.

21. Verfahren zur Markierung und Reinigung von Nukleinsäuren von Interesse, die in einer zu behandelnden biologischen Probe vorliegen, das folgendes umfaßt:
• Verfügen über ein einziges Reaktionsgefäß,
• Einbringen in dieses Reaktionsgefäß von folgendem:
- die biologische Probe,
- mindestens einen Reaktanten für die Markierung von Nukleinsäuren, wobei man den Markierungsreaktanten mit Nukleinsäuren in homogener Lösung in einem im wesentlichen wäßrigen Puffer in Kontakt bringt, wobei dieser Markierungsreaktant temperaturstabil ist und die Formel (6) aufweist: 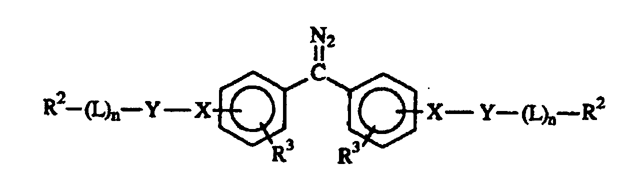 in der
• R² einen nachweisbaren Marker oder mindestens zwei nachweisbare Marker, die untereinander mittels mindestens einer multimeren Struktur verbunden sind, bedeutet,
• R³ H, NO₂, Cl, Br, F, I, R²-(L)ₙ-Y-X-, OR, SR, NR₂, R, NHCOR, CONHR, COOR, wobei R = Alkyl oder Aryl, bedeutet,
• L ein Bindeglied bedeutet, das eine lineare Verknüpfung von mindestens zwei kovalenten Bindungen enthält, und n eine ganze Zahl gleich 0 oder 1 bedeutet, und
• -Y-X- -CONH-, -NHCO-, -CH₂O-, -CH₂S-bedeutet,
- mindestens einen festen Träger, der die Adsorption der genannten Nukleinsäuren ermöglicht,
- beliebige Bestandteile, die für die Markierung der Nukleinsäuren und/oder die Immobilisierung der genannten Nukleinsäuren auf dem Träger erforderlich sind,
• Inkubieren des Inhalts des Reaktionsgefäßes, und
• Isolieren der so markierten Nukleinsäuren.

22. Verfahren zur Markierung und Reinigung von Nukleinsäuren von Interesse, die in einer zu behandelnden biologischen Probe vorliegen, das folgendes umfaßt:
• Verfügen über ein einziges Reaktionsgefäß,
• Einbringen in dieses Reaktionsgefäß von folgendem:
- die biologische Probe,
- mindestens einen Reaktanten für die Markierung von Nukleinsäuren, wobei man den Markierungsreaktanten mit Nukleinsäuren in homogener Lösung in einem im wesentlichen wäßrigen Puffer in Kontakt bringt, wobei dieser Markierungsreaktant temperaturstabil ist und die Formel (25) aufweist: 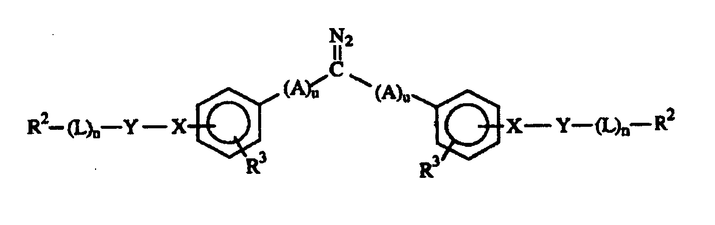 in der
• R² einen nachweisbaren Marker oder mindestens zwei nachweisbare Marker, die untereinander mittels mindestens einer multimeren Struktur verbunden sind, bedeutet,
• R³ H, NO₂, Cl, Br, F, I, R²-(L)ₙ-Y-X-, OR, SR, NR₂, R, NHCOR, CONHR, COOR, wobei R = Alkyl oder Aryl, bedeutet,
• L ein Bindeglied bedeutet, das eine lineare Verknüpfung von mindestens zwei kovalenten Bindungen enthält, und n eine ganze Zahl gleich 0 oder 1 bedeutet,
• A ein Bindeglied bedeutet, das mindestens eine kovalente Doppelbindung enthält, die die Konjugation der Diazofunktion mit dem aromatischen Ring gestattet, und u gleich 1 ist, und
• -Y-X- -CONH-, -NHCO-, -CH₂O-, -CH₂S-bedeutet,
- mindestens einen festen Träger, der die Adsorption der genannten Nukleinsäuren ermöglicht,
- beliebige Bestandteile, die für die Markierung der Nukleinsäuren und/oder die Immobilisierung der genannten Nukleinsäuren auf dem Träger erforderlich sind,
• Inkubieren des Inhalts des Reaktionsgefäßes, und
• Isolieren der so markierten Nukleinsäuren.

23. Verfahren nach einem der Ansprüche 9 bis 11, bei dem der Reaktant die Formel (14) aufweist: 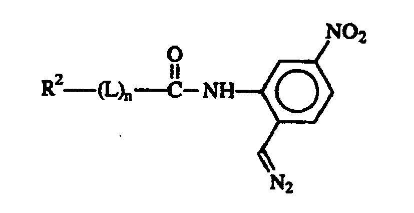 in der
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet.

24. Verfahren nach einem der Ansprüche 9 oder 14, bei dem der Reaktant die Formel (26) aufweist: 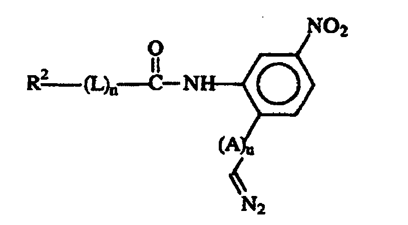 in der
• R² einen nachweisbaren Marker oder mindestens zwei nachweisbare Marker, die untereinander mittels mindestens einer multimeren Struktur verbunden sind, bedeutet,
• A ein Bindeglied bedeutet, das mindestens eine kovalente Doppelbindung enthält, die die Konjugation der Diazofunktion mit dem aromatischen Ring gestattet, und u gleich 1 ist, und
• L ein Bindeglied bedeutet, das eine lineare Verknüpfung von mindestens zwei kovalenten Bindungen enthält, und n eine ganze Zahl gleich 0 oder 1 bedeutet.

25. Verfahren nach einem der Ansprüche 9 bis 11, bei dem der Reaktant die Formel (15) aufweist: 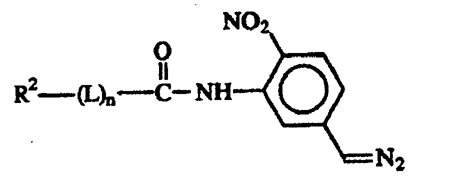 in der
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet.

26. Verfahren nach einem der Ansprüche 9 oder 14, bei dem der Reaktant die Formel (27) aufweist: 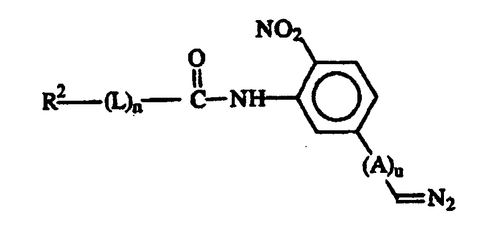 in der
• R² einen nachweisbaren Marker oder mindestens zwei nachweisbare Marker, die untereinander mittels einer multimeren Struktur verbunden sind, bedeutet,
• A ein Bindeglied bedeutet, das mindestens eine kovalente Doppelbindung enthält, die die Konjugation der Diazofunktion mit dem aromatischen Ring gestattet, und u gleich 1 ist, und
• L ein Bindeglied bedeutet, das eine lineare Verknüpfung von mindestens zwei kovalenten Bindungen enthält, und n eine ganze Zahl gleich 0 oder 1 bedeutet.

27. Verfahren nach einem der Ansprüche 9 bis 26, **dadurch gekennzeichnet, daß** der Bestandteil L des Reaktanten ein 1- bis 20mal, vorzugsweise 1- bis 10mal, noch stärker bevorzugt 2- bis 5mal wiederholtes Motiv -(O-CH₂-CH₂)- enthält.

28. Verfahren zur Markierung und Reinigung von Nukleinsäuren von Interesse, die in einer zu behandelnden biologischen Probe vorliegen, das folgendes umfaßt:
• Verfügen über ein einziges Reaktionsgefäß,
• Einbringen in dieses Reaktionsgefäß von folgendem:
- die biologische Probe,
- mindestens einen Reaktanten für die Markierung von Nukleinsäuren, wobei der Markierungsreaktant die Markierung und Fragmentierung einer einzelsträngigen oder doppelsträngigen Nukleinsäure nach den folgenden Schritten ermöglicht:
? Fragmentierung der Nukleinsäure,
? Anbringen eines Markers an mindestens einem der Fragmente mittels eines Markierungsreaklanten ausgewählt aus den Verbindungen der Formel (19): 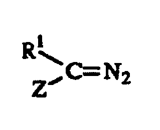 in der
• R¹ H oder eine Gruppe Alkyl, Aryl oder substituiertes Aryl bedeutet, und
• Z einen nachweisbaren Marker umfaßt,
wobei dieser Reaktant kovalent und hauptsächlich mit mindestens einem Phosphat dieses Fragments koppelt,
- mindestens einen festen Träger, der die Adsorption der genannten Nukleinsäuren ermöglicht,
- beliebige Bestandteile, die für die Markierung der Nukleinsäuren und/oder die Immobilisierung der genannten Nukleinsäuren auf dem Träger erforderlich sind,
• Inkubieren des Inhalts des Reaktionsgefäßes, und
• Isolieren der so markierten Nukleinsäuren.

29. Verfahren nach Anspruch 26, **dadurch gekennzeichnet, daß** der Markierungsreaktant aus den Verbindungen der Formel (20) ausgewählt wird: 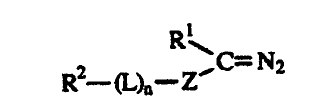 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet, und
• Z aus der folgenden Gruppe stammt: 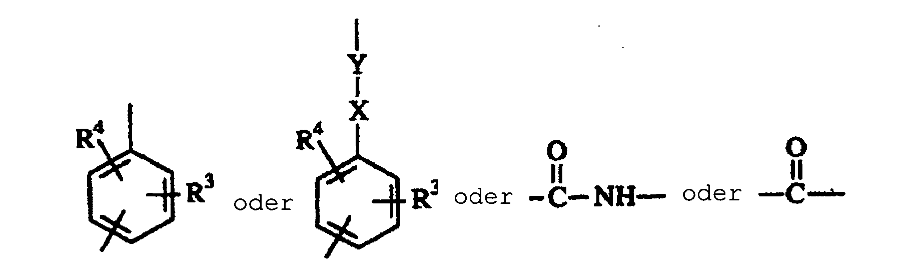
worin:
■ R³ und R⁴ unabhängig voneinander folgendes bedeuten: H, NO₂, Cl, Br, F, I, R² - (L)ₙ-Y-X-, OR, SR, NR₂, R, NHCOR, CONHR, COOR, wobei R = Alkyl oder Aryl, und
■ -Y-X- folgendes bedeutet: -CONH-, -NHCO-, -CH₂O-, -CH₂S-.

30. Verfahren nach Anspruch 28, **dadurch gekennzeichnet, daß** Z 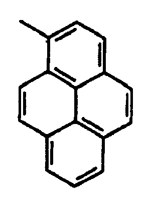 bedeutet.

31. Verfahren nach einem der Ansprüche 28 bis 30, **dadurch gekennzeichnet, daß** die Fragmentierung und die Markierung in zwei Schritten erfolgen.

32. Verfahren nach einem der Ansprüche 28 bis 30, **dadurch gekennzeichnet, daß** die Fragmentierung und die Markierung in einem Schritt erfolgen.

33. Verfahren nach einem der Ansprüche 28 bis 32, **dadurch gekennzeichnet, daß** die Markierung in homogener, im wesentlichen wäßriger Lösung erfolgt.

34. Verfahren nach einem der Ansprüche 28 bis 33, **dadurch gekennzeichnet, daß** die Fragmentierung auf enzymatischem, physikalischem oder chemischem Weg erfolgt.

35. Verfahren zur Markierung und Reinigung von Nukleinsäuren von Interesse, die in einer zu behandelnden biologischen Probe vorliegen, das folgendes umfaßt:
• Verfügen über ein einziges Reaktionsgefäß,
• Einbringen in dieses Reaktionsgefäß von folgendem:
- die biologische Probe,
- mindestens einen Reaktanten für die Markierung von Nukleinsäuren, wobei man den Markierungsreaktanten in homogener Lösung in einem im wesentlichen wäßrigen Puffer in Kontakt bringt, wobei dieser Markierungsreaktant temperaturstabil ist und die Formel (8) aufweist: 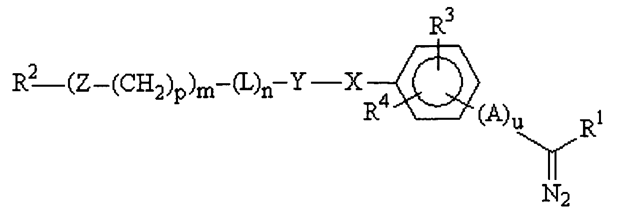 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet,
• R³ und R⁴ unabhängig voneinander H, NO₂, Cl, Br, F, I, R² - (L)ₙ-Y-X-, OR, SR, NR₂, R, NHCOR, CONHR, COOR, -CO-NH-(CH₂)₃-(O-CH₂-CH₂) ₃-CH₂-NH-R², -CO-NH- (CH₂)₃- (O-CH₂-CH₂)₄-CH₂-NH-R², wobei R = Alkyl oder Aryl, bedeuten,
• A ein Bindeglied bedeutet, das mindestens eine kovalente Doppelbindung enthält, die die Konjugation der Diazofunktion mit dem aromatischen Ring gestattet, und u eine ganze Zahl zwischen 0 und 2 bedeutet und vorzugsweise 0 oder 1 ist, und
• -Y-X- -CONH-, -NHCO-, -CH₂O- -CH₂S- bedeutet,
• -Z- -NH-, -NHCO-, -CONH- oder -0- bedeutet,
• m eine ganze Zahl zwischen 1 und 10, vorzugsweise zwischen 1 und 3, bedeutet, und
• p eine ganze Zahl zwischen 1 und 10, vorzugsweise zwischen 1 und 3, bedeutet,
- mindestens einen festen Träger, der die Adsorption der genannten Nukleinsäuren ermöglicht,
- beliebige Bestandteile, die für die Markierung der Nukleinsäuren und/oder die Immobilisierung der genannten Nukleinsäuren auf dem Träger erforderlich sind,
• Inkubieren des Inhalts des Reaktionsgefäßes, und
• Isolieren der so markierten Nukleinsäuren.

36. Verfahren nach Anspruch 35, bei dem der Reaktant die Formel (9) aufweist: 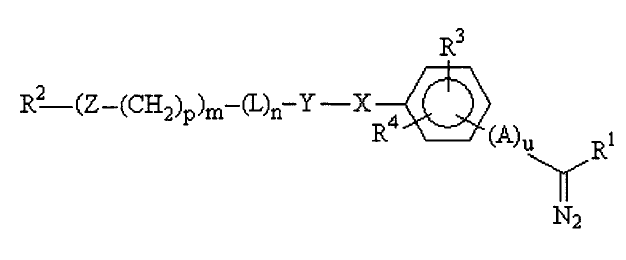 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet,
• R³ und R⁴ unabhängig voneinander H, NO₂, Cl, Br, F, I, R² - (L)ₙ-Y-X-, OR, SR, NR₂, R, NHCOR, CONHR, COOR, -CO-NH-(CH₂)₃-(O-CH₂- CH₂)₃-CH₂-NH-R², -CO-NH- (CH₂)₃-(O-CH₂-CH₂)₄-CH₂-NH-R², wobei R = Alkyl oder Aryl, bedeuten,
• A ein Bindeglied bedeutet, das mindestens eine kovalente Doppelbindung enthält, die die Konjugation der Diazofunktion mit dem aromatischen Ring gestattet, und u eine ganze Zahl zwischen 0 und 2 bedeutet und vorzugsweise 0 oder 1 ist,
• -Y-X- -CONH-, -NHCO-, -CH₂O-, -CH₂S- bedeutet,
• m eine ganze Zahl zwischen 1 und 10, vorzugsweise zwischen 1 und 3, bedeutet, und
• p eine ganze Zahl zwischen 1 und 10, vorzugsweise zwischen 1 und 3, bedeutet.

37. Verfahren nach einem der Ansprüche 35 oder 36, **dadurch gekennzeichnet, daß** in der Formel des Reaktanten p gleich oder kleiner m ist.

38. Verfahren nach einem der Ansprüche 35 bis 37, bei dem der Reaktant die Formel (10) aufweist: 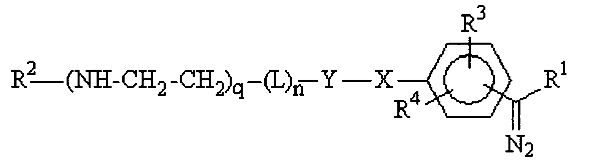 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet,
• R³ und R⁴ unabhängig voneinander H, NO₂, Cl, Br, F, I, R² - (L)ₙ-Y-X-, OR, SR, NR₂, R, NHCOR, CONHR, COOR, -CO-NH-(CH₂)₃-(O-CH₂- CH₂)₃-CH₂-NH-R², -CO-NH-(CH₂)₃-(O-CH₂-CH₂)₄-CH₂-NH-R², wobei R = Alkyl oder Aryl, bedeuten, und
• q eine ganze Zahl zwischen 1 und 10, vorzugsweise zwischen 1 und 3, bedeutet.

39. Verfahren nach einem der Ansprüche 35 bis 38, bei dem der Reaktant die Formel (11) aufweist: 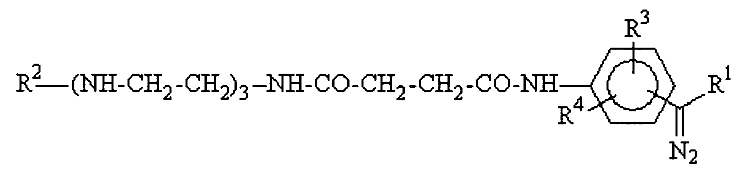 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet, und
• R³ und R⁴ unabhängig voneinander H, NO₂, Cl, Br, F, I, R² - (L)ₙ-Y-X-, OR, SR, NR₂, R, NHCOR, CONHR, COOR, -CO-NH-(CH₂)₃-(O-CH₂- CH₂)₃-CH₂-NH-R², -CO-NH-(CH₂)₃- (O-CH₂-CH₂)₄CH₂-NH-R², wobei R = Alkyl oder Aryl, bedeuten.

40. Verfahren nach einem der Ansprüche 35 bis 39, **dadurch gekennzeichnet, daß** R² in dem Reaktanten aus einem D-Biotinrest der Formel (12) besteht:

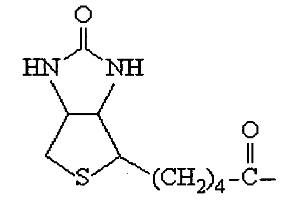

41. Verfahren nach einem der Ansprüche 35 bis 40, **dadurch gekennzeichnet, daß** R¹ in dem Reaktanten aus CH₃ besteht und R³ und R⁴ jeweils H bedeuten.

42. Verfahren nach einem der Ansprüche 35 bis 41, bei dem die Struktur -(L)ₙ- des Reaktanten aus folgendem besteht:
• Spermin oder N,N'-Bis(3-aminopropyl)-1,4-diaminobutan: NH₂- (CH₂)₃-NH- (CH₂)₄-NH- (CH₂)₃-NH₂, oder
• Spermidin oder N-(3-Aminopropyl)-1,4-butandiamin: H₂N-(CH₂)₄-NH-(CH₂)₃-NH₂, oder
• einem Derivat, das ein Alanin-Motiv: NH₂-CH₂-CH₂-COOH enthält.

43. Verfahren zur Markierung und Reinigung von Nukleinsäuren von Interesse, die in einer zu behandelnden biologischen Probe vorliegen, das folgendes umfaßt:
• Verfügen über ein einziges Reaktionsgefäß,
• Einbringen in dieses Reaktionsgefäß von folgendem:
- die biologische Probe,
- mindestens einen Reaktanten für die Markierung von Nukleinsäuren, wobei man den Markierungsreaktanten in homogener Lösung in einem wäßrigen Puffer in Kontakt bringt, wobei der Markierungsreaktant die Formel (13) aufweist: 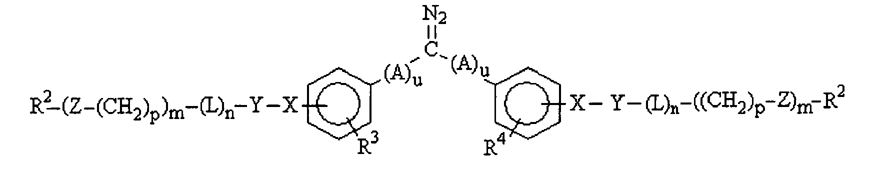 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet,
• R³ und R⁴ unabhängig voneinander H, NO₂, Cl, Br, F, I, R² - (L)ₙ-Y-X-, OR, SR, NR₂, R, NHCOR, CONHR, COOR, -CO-NH-(CH₂)₃-(O-CH₂-CH₂)₃-CH₂-NH-R², -CO-NH-(CH₂)₃-(O-CH₂-CH₂)₄-CH₂-NH-R², wobei R = Alkyl oder Aryl, bedeuten,
• A ein Bindeglied bedeutet, das mindestens eine kovalente Doppelbindung enthält, die die Konjugation der Diazofunktion mit dem aromatischen Ring gestattet, und u eine ganze Zahl zwischen 0 und 2 bedeutet und vorzugsweise 0 oder 1 ist, und
• -Y-X- -CONH-, -NHCO-, -CH₂O-, -CH₂S- bedeutet,
• -Z- -NH-, -NHCO-, -CONH- oder -0- bedeutet,
• m eine ganze Zahl zwischen 1 und 10, vorzugsweise zwischen 1 und 3, bedeutet, und
• p eine ganze Zahl zwischen 1 und 10, vorzugsweise zwischen 1 und 3, bedeutet,
- mindestens einen festen Träger, der die Adsorption der genannten Nukleinsäuren ermöglicht,
- beliebige Bestandteile, die für die Markierung der Nukleinsäuren und/oder die Immobilisierung der genannten Nukleinsäuren auf dem Träger erforderlich sind,
• Inkubieren des Inhalts des Reaktionsgefäßes, und
• Isolieren der so markierten Nukleinsäuren.

44. Verfahren nach Anspruch 43, bei dem der Reaktant die Formel (16) aufweist: 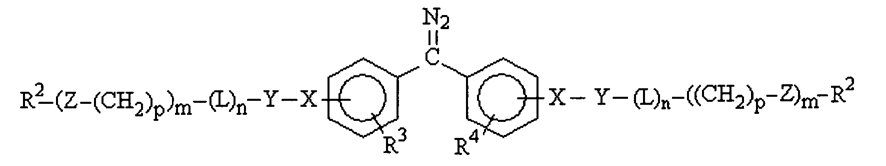 in der
• R¹ Wasserstoff oder eine Gruppe Alkyl, Aryl oder substituiertes Aryl bedeutet,
• R² einen nachweisbaren Marker oder mindestens zwei nachweisbare Marker, die untereinander mittels mindestens einer multimeren Struktur verbunden sind, bedeutet,
• L ein Bindeglied bedeutet, das eine lineare Verknüpfung von mindestens zwei kovalenten Bindungen enthält, und n eine ganze Zahl gleich 0 oder 1 bedeutet,
• R³ und R⁹ unabhängig voneinander H, NO₂, Cl, Br, F, I, R² - (L)ₙ-Y-X-, OR, SR, NR₂, R, NHCOR, CONHR, COOR, -CO-NH-(CH₂)₃-(O-CH₂-CH₂)₃-CH₂-NH-R², -CO-NH-(CH₂)₃-(O-CH₂-CH₂)₄-CH₂-NH-R², wobei R = Alkyl oder Aryl, bedeuten,
• -Y-X- -CONH-, -NHCO-, -CH₂O-, -CH₂S- bedeutet,
• -Z- -NH-, -NHCO-, -CONH- oder -O- bedeutet,
• m eine ganze Zahl zwischen 1 und 10, vorzugsweise zwischen 1 und 3, bedeutet, und
• p eine ganze Zahl zwischen 1 und 10, vorzugsweise zwischen 1 und 3, bedeutet.

45. Verfahren nach einem der Ansprüche 35 bis 44, **dadurch gekennzeichnet, daß** der Bestandteil L des Reaktanten ein 1- bis 20mal, vorzugsweise 1- bis 10mal, noch stärker bevorzugt 2- bis 5mal wiederholtes Motiv -(O-CH₂-CH₂)- enthält, wobei -Z-NH-, -NHCO- oder -CONH- bedeutet.

46. Verfahren nach einem der Ansprüche 1 bis 45, **dadurch gekennzeichnet, daß** der feste Träger aus Silicateilchen besteht.

47. Verfahren nach einem der Ansprüche 1 bis 46, **dadurch gekennzeichnet, daß** der feste Träger aus silicabeschichteten magnetischen Teilchen besteht.

48. Verfahren nach einem der Ansprüche 46 bis 47, **dadurch gekennzeichnet, daß** die Silicateilchen, die den festen Träger bilden, Teilchengrößen zwischen 0,1 und 500 µm, vorzugsweise zwischen 1 und 200 µm, aufweisen.

49. Verfahren nach einem der Ansprüche 1 bis 48, **dadurch gekennzeichnet, daß** einer der zusätzlichen Bestandteile, die die Markierung ermöglichen, aus einem Alkohol, vorzugsweise Isopropanol, bestehen.

50. Verfahren nach Anspruch 49, **dadurch gekennzeichnet, daß** das Isopropanol 70% (v/v) der zum Ende erhaltenen Mischung ausmacht.

51. Verfahren nach einem der Ansprüche 1 bis 50, **dadurch gekennzeichnet, daß** die zusätzlichen Bestandteile, die die Freisetzung aus den Zellen und daher die Adsorption der Nukleinsäuren auf dem festen Träger ermöglichen, aus einem chaotropen Mittel bestehen.

52. Verfahren nach Anspruch 51, **dadurch gekennzeichnet, daß** es sich bei dem chaotropen Mittel um ein Guanidiumsalz, Natriumiodid, Kaliumiodid, Natrium(iso)thiocyanat, Harnstoff oder Mischungen dieser Derivate handelt.

53. Verfahren nach Anspruch 52, **dadurch gekennzeichnet, daß** es sich bei dem verwendeten Guanidiumsalz um Guanidium(iso)thiocyanat handelt.

54. Verfahren nach einem der Ansprüche 1 bis 53, **dadurch gekennzeichnet, daß** man die Festphasen-Nukleinsäure-Komplexe von den Flüssigkeiten durch Sedimentation und Verwerfen des Überstands sowie anschließendes Waschen der Komplexe mit einem Waschpuffer, der eine chaotrope Substanz enthält, trennt.

55. Verfahren nach Anspruch 54, **dadurch gekennzeichnet, daß** die Festphasen-Nukleinsäuren-Komplexe, die mit dem Waschpuffer gewaschen wurden, noch zusätzlich mit Hilfe von einem oder mehreren organischen Lösungsmitteln gewaschen und anschließend getrocknet werden.

56. Verfahren nach Anspruch 55, **dadurch gekennzeichnet, daß** die in den gewaschenen und getrockneten Festphasen-Nukleinsäure-Komplexen vorliegende Nukleinsäure mit Hilfe eines Elutionspuffers eluiert wird.

57. Verfahren nach einem der Ansprüche 1 bis 53, **dadurch gekennzeichnet, daß** die so erhaltenen Festphasen-Nukleinsäure-Komplexe mit einer Mischung, in der die Bestandteile vorliegen, im Hinblick auf eine Amplifikation der Nukleinsäure, die entweder an besagte Festphase gebunden ist oder von dieser eluiert worden ist, in Kontakt gebracht werden.

58. Verfahren nach einem der Ansprüche 1 bis 57, **dadurch gekennzeichnet, daß** der Inkubationsschritt darin besteht, daß man die behandelte Probe 5 bis 45 Minuten lang, vorzugsweise 15 bis 35 Minuten lang, noch stärker bevorzugt 25 Minuten lang, bei einer Temperatur zwischen 45 und 85°C, vorzugsweise zwischen 55 bis 75°C, noch stärker bevorzugt bei 65°C, hält.

59. Verfahren nach Anspruch 58, **dadurch gekennzeichnet, daß** die Probe nach dem Inkubationsschritt mindestens einige Minuten lang, vorzugsweise 5 Minuten lang, auf Raumtemperatur gebracht wird.

60. Markierungs- und Reinigungsverfahren nach einem der Ansprüche 21, 22, 28, 35 oder 43, **dadurch gekennzeichnet, daß** die behandelten Nukleinsäuren aus einzelsträngigen und/oder doppelsträngigen, synthetischen und/oder natürlichen DNAs und/oder RNAs bestehen.
